# EUROPEAN PATENT SPECIFICATION

(11) **EP 1 332 214 B1**
(45) Date of publication and mention of the grant of the patent: **18.11.2009**
(21) Application number: 01979491.6
(22) Date of filing: 27.09.2001
(51) Int. Cl.: C12N 15/12, C12N 15/55, C12N 9/78, C07K 16/40, C12Q 1/68, G01N 33/53, A61P 35/00

(54) **32144, A HUMAN FATTY ACID AMIDE HYDROLASE AND USES THEREOF**
32144, EINE HUMANE FETTSÄUREAMID-HYDROLASE UND DEREN VERWENDUNGEN
32144, MEMBRE DE LA FAMILLE DES AMIDES HYDROLASES D'ACIDE GRAS HUMAINES ET SES UTILISATIONS

(30) Priority: 05.10.2000 US 238054 P
(43) Date of publication of application: 06.08.2003
(73) Proprietor: MILLENNIUM PHARMACEUTICALS, INC., Cambridge, MA 02139-4169 (US)
(72) Inventor: CURTIS, Rory, A., J., Southborough, MA 01772 (US); MACBETH, Kyle, J., Boston, MA 02118 (US); RUDOLPH-OWEN, Laura, A., Jamaica Plain, MA 02130 (US)
(74) Representative: Taylor, Kate Laura
(86) International application number: PCT/US2001/031188
(87) International publication number: WO 2002/029057

(56) References cited:
- WO-A-01/02568
- WO-A-01/51628
- WO-A-01/83524
- DATABASE EMBL, HEIDELBERG, FRG [Online] 13 September 2000 (2000-09-13) NCI-CGAP: "7e55c12.x1 NCI_CGAP_Lu24 Homo sapiens cDNA clone IMAGE: 3286390 3' similar to WP: Y53F4B.W CE22408, mRNA sequence" Database accession no. BE671210 XP002210156 cited in the application
- DATABASE EMBL, HEIDELBERG, FRG [Online] derived from Caenorhabditis elegans, 1 October 2000 (2000-10-01) SMYE, R.: "Y53F4B.18 protein" Database accession no. Q9NAB7 XP002210157 -& DATABASE EMBL, HEIDELBERG, FRG [Online] 12 November 1999 (1999-11-12) SMYE, R.: "Caenorhabditis elegans cosmid Y53F4B" Database accession no. AL132949 XP002210158 -& THE C. ELEGANS SEQUENCING CONSORTIUM: "Genome Sequence of the Nematode C. elegans: A Platform for Investigating Biology" SCIENCE, vol. 282, no. 5396, 11 December 1998 (1998-12-11), pages 2012-2018, XP002199976
- DATABASE EMBL, HEIDELBERG, FRG [Online] 8 July 1999 (1999-07-08) NCI-CGAP: "tr03d06.x1 NCI_CGAP_Ov23 Homo sapiens cDNA clone IMAGE: 2217227 3', mRNA sequence" Database accession no. AI811051 XP002210159 cited in the application
- BISOGNO, T. ET AL.: "Biosynthesis and degradation of biactive fatty acid amides in human breast cancer and rat pheochromocytoma cells" EUROPEAN JOURNAL OF BIOCHEMISTRY, vol. 254, no. 3, 15 June 1998 (1998-06-15), pages 634-642, XP002210154
- DI MARZO, V. ET AL.: "Palmitoylethanolamide inhibits the expression of fatty acid amide hydrolase and enhances the anti-proliferative effect of anandamide in human breast cancer cells" BIOCHEMICAL JOURNAL, vol. 358, no. 1, 15 August 2001 (2001-08-15), pages 249-255, XP002210155

## Description

### Background of the Invention

Fatty acid amides represent a growing family of bioactive lipids with diverse cellular and physiological effects (Cravatt, B.F. et al. (1995), Science 268:1506-1509; Devane, W.A. et al. (1992), Science 258:1946-1949; Facci, L. et al. (1995), Proc. Natl. Acad. Sci. 92:3376-3380). Members of this family include oleamide, a sleep-inducing lipid, and anandamide, an endogenous ligand in the brain CB1 cannabinoid receptor (Cravatt, B.F. et al. (1995), supra; Cravatt, B.F. et al. (1996), J. Am. Chem. Soc. 118:580-590).

In addition to its sleep-inducing properties, oleamide has been shown to potentiate the response of 5-HT₂ receptors to serotonin (Huidobro-Toro, J. et al. (1996), Proc. Natl. Acad, Sci, 93:8078-8082). Anandamide has been shown to have analgesic effects in rats (Devane, W.A. et al. (1992), supra) and, at a cellular and molecular level, has been demonstrated to regulate focal adhesion kinase activity in hippocampal slices (Derkinderen, P. et al. (1996), Science 273:1719-1722) and to inhibit prolactin and/or nerve growth factor-induced cell proliferation in human breast cancer cell lines (DiMarzo et al. (2000), Prostaglandins Other Lipid Mediat, 61(1-2):43-61; DePetrocellis (1998), Proc Natl Acad Sci 95(14):8375-80). In addition, anandamide inhibits the 5-HT₃ ion channel in rat nodose ganglion neurons (Fan, P. (1995), J. Neurophysiol. 73:907-910) and arrests development of preimplantation mouse embryos (Paria, B. C. et al. (1995), Proc. Natl. Acad Sci. 92:9460-9464), and both oleamide and anandamide have been found to block gap junction communication in glial cells (Venance, L. et al. (1995), Nature 376:590-594). Other fatty acid amides have been reported to possess biological activities, as well (Facci, L. et al. (1995), supra). In particular, palmitoyl ethanolamide has been shown to bind selectively over anandamide to the peripheral CB2 cannabinoid receptor, indicating perhaps that the CB1 and CB2 receptors recognize distinct fatty acid amides as their respective lignads (Barg, J. et al. (1995), Eur. J. Pharmacol. 287: 145-152; Skaper, S.D. et al. (1996), Proc. Natl. Acad Sci. 93:3484-3989).

The neurophysiological effects of both oleamide and anandamide, in conjunction with the isolation of these compounds from cerebrospinal fluid and brain tissue, respectively, suggest that fatty acid amides may serve important neuromodulatory roles in the central nervous system. Likewise, the effects of anandamide on cellular proliferation and focal adhesion kinase activity suggests that fatty acid amides may be important anti-cancer agents.

Fatty acid amides are hydrolyzed by enzymes known as fatty acid amide hydrolases (Ueda et al. (2000), Chem Phys Lipids 108(1-2):107-21). Fatty acid amide hydrolases (FAAHs) have been cloned from rat liver plasma and demonstrated to hydrolyze both oleamide and anandamide, as well as several other fatty acid amides (Cravatt et al. (1996), Nature 384:83-7). In situ hybridization studies have shown prominent expression of FAAH in a variety of rat neuronal cells. Human and mouse fatty acid amide hydrolases have also been reported (Giang, D.K. and Cravatt (1997), Proc. Natl. Acad. Sci. 94:2238-2242.

WO 0151628 provides novel markers, wherein changes in the levels of expression of one or more of the markers is correlated with the presence of breast cancer. WO 0183524 provides methods for diagnosing, treating, or preventing disorders associated with aberrant expression of human RNA metabolism proteins (RMEP).

Given the critical role of FAAHs in fatty acid amide metabolism, there exists a need for identifying and characterizing novel members of this enzyme family.

### Summary of the Invention

The present invention is based, in part, on the discovery of a novel fatty acid amide hydrolase family member, referred to herein as "32144". The nucleotide sequence of a cDNA encoding 32144 is shown in SEQ ID NO: 1, and the amino acid sequence of a 32144 polypeptide is shown in SEQ ID NO:2. In addition, the nucleotide sequences of the coding region are depicted in SEQ ID NO:3.

In one aspect the invention provides a method for identifying a subject having a lung, colon or ovarian cancer, or at risk of developing a lung, colon or ovarian cancer, the method comprising: a) contacting a first sample obtained from said subject comprising nucleic acid molecules with a nucleic acid probe that specifically hybridizes to a nucleic acid molecule of SEQ ID NO: 1 or SEQ ID NO:3 as shown in example 1; b) detecting the presence of a nucleic acid molecule in the first sample that specifically hybridizes to the probe; c) contacting a second sample from a control subject comprising nucleic acid molecules with a nucleic acid probe which specifically hybridizes to a nucleic acid molecule of SEQ ID NO: 1 or SEQ ID NO:3 as shown in example 1; d) detecting the presence of a nucleic acid molecule in the second sample that specifically hybridizes to the probe; e) comparing the level of nucleic acids which hybridise to the probe in the first sample with the level of nucleic acids which hybridize to the probe in the second sample, wherein an increased level of nucleic acids which hybridise in the first sample relative to the second sample identifies a subject having a lung, colon or ovarian cancer, or at risk of developing a lung, colon or ovarian cancer.

In one embodiment said hybridization probe is detectably labeled. In one embodiment said detecting is by *in situ* hybridization.

In a further aspect the invention provides a method for identifying a subject having a lung, colon or ovarian cancer, or at risk of developing a lung, colon or ovarian cancer, the method comprising: a) contacting a first sample obtained from said subject comprising nucleic acid molecules with a first and a second amplification primer, each of which specifically hybridizes to a nucleic acid molecule selected from the group consisting of: i) a nucleic acid molecule consisting of the nucleotide sequence set forth in SEQ ID NO:1 or 3 as shown in example 1; and ii) a nucleic acid molecule which encodes a polypeptide comprising the amino acid sequence set forth in SEQ ID NO:2 as shown in example 1; b) incubating the first sample under conditions that allow nucleic acid amplification; c) contacting a second sample obtained from a control subject comprising nucleic acid molecules with said first and a second amplification primer; d) incubating the second sample under conditions that allow nucleic acid amplification; and e)comparing the level of nucleic acid amplification in the first sample relative to the level of nucleic acid amplification in the second sample, wherein an increased level of nucleic acid amplification in the first sample relative to the second sample identifies a subject having a lung, colon or ovarian cancer, or at risk of developing a lung, colon or ovarian cancer.

In a further aspect the invention provides a method of identifying a subject having a lung, colon or ovarian cancer, or at risk of developing a lung, colon or ovarian cancer comprising:
a) contacting a first sample obtained from said subject comprising polypeptides with an antibody which specifically binds to a polypeptide selected from the group consisting of: i) a polypeptide comprising the amino acid sequence of SEQ ID NO:2 as shown in example 1; and ii) a polypeptide which is encoded by a nucleic acid molecule comprising the nucleotide sequence of SEQ ID NO:1 or 3 as shown in example 1; b)detecting the presence of a polypeptide in the first sample that binds to the antibody; c) contacting a second sample from a control subject comprising polypeptides with the antibody; d) detecting the presence of a polypeptide in the second sample that binds to the antibody; and e) comparing the level of antibody binding in the first sample relative to the level of antibody binding in the second sample, wherein an increased level of antibody binding in the first sample relative to the second sample identifies a subject having a lung, colon or ovarian cancer, or at risk of developing a lung, colon or ovarian cancer.

In a preferred embodiment said antibody is detectably labeled.

In a further aspect the invention provides a method for identifying a compound capable of treating a lung, colon or ovarian cancer, the method comprising: assaying the ability of the compound to decrease expression of a nucleic acid molecule selected from the group consisting of: i) a nucleic acid molecule comprising a nucleotide sequence which is at least 95% identical to the nucleotide sequence of SEQ ID NO:1 or 3 as shown in example 1, or a complement thereof; ii) a nucleic acid molecule which encodes a polypeptide comprising an amino acid sequence which is at least 95% identical to the amino acid sequence of SEQ ID NO:2 as shown in example 1; iii) a nucleic acid molecule consisting of the nucleotide sequence set forth in SEQ ID NO:1 or 3 as shown in example 1; and iv) a nucleic acid molecule which encodes a polypeptide comprising the amino acid sequence set forth in SEQ ID NO:2 as shown in example 1; thereby identifying a compound capable of treating lung, colon or ovarian cancer.

In a further aspect the invention provides a method for identifying a compound capable of treating a lung, colon or ovarian cancer, the method comprising: assaying the ability of the compound to decrease the fatty acid amide hydrolase activity of a polypeptide selected from the group consisting of: i) a polypeptide which is encoded by a nucleic acid molecule comprising a nucleotide sequence which is at least 95% identical to a nucleic acid comprising the nucleotide sequence of SEQ ID NO:1 or 3 as shown in example 1; ii) a polypeptide comprising an amino acid sequence which is at least 95% identical to the amino acid sequence of SEQ ID NO:2 as shown in example 1; iii) a polypeptide comprising the amino acid sequence of SEQ ID NO:2 as shown in example 1; and iv) a polypeptide which is encoded by a nucleic acid molecule comprising the nucleotide sequence of SEQ ID NO:1 or 3 as shown in example 1; thereby identifying a compound capable of treating lung, colon or ovarian cancer.

In a one embodiment said assay is a cell based assay. In one embodiment said assay is a cell free assay.

In a further aspect the invention provides a method for evaluating the efficacy of a treatment of a lung, colon or ovarian cancer, in a sample isolated from a subject being treated with a protocol under evaluation, which comprises: assessing the expression level of a nucleic acid molecule selected from the group consisting of: a) a nucleic acid molecule comprising a nucleotide sequence which is at least 95% identical to the nucleotide sequence of SEQ ID NO:1 or SEQ ID NO:3 as shown in example 1; b) a nucleic acid molecule which encodes a polypeptide comprising the amino acid sequence of SEQ ID NO:2 as shown in example 1; and c) a nucleic acid molecule comprising the nucleotide sequence of SEQ ID NO:1 or SEQ ID NO:3 as shown in example 1; wherein a decrease in the expression level of the nucleic acid after the treatment, relative to the level before the treatment, is indicative of the efficacy of the treatment of a lung, colon or ovarian cancer.

In a further aspect the invention provides a method for evaluating the efficacy of a treatment of a lung, colon or ovarian cancer, in a sample isolated from a subject being treated with a protocol under evaluation, which comprises: assessing the expression level of a polypeptide selected from the group consisting of: i) a polypeptide which is encoded by a nucleic acid molecule comprising a nucleotide sequence which is at least 95% identical to a nucleic acid comprising the nucleotide sequence of SEQ ID NO:1 or 3 as shown in example 1; ii) a polypeptide comprising an amino acid sequence which is at least 95% identical to the amino acid sequence of SEQ ID NO:2 as shown in example 1; iii) a polypeptide comprising the amino acid sequence of SEQ ID NO:2 as shown in example 1; and iv) a polypeptide which is encoded by a nucleic acid molecule comprising the nucleotide sequence of SEQ ID NO:1 or 3 as shown in example 1; wherein a decrease in the expression level of the polypeptide after the treatment, relative to the level before the treatment, is indicative of the efficacy of the treatment of a lung, colon or ovarian cancer.

The invention features use of 32144 polypeptides or nucleic acids, e.g., as reagents or targets in assays applicable to treatment and diagnosis of 32144-mediated or -related disorders, e.g., cellular proliferative disorders of the lung, colon, or ovary.

The invention provides methods of screening for compounds that modulate the expression or activity of the 32144 polypeptides or nucleic acids.

The invention provides methods for evaluating the efficacy of a treatment of a disorder, e.g., a proliferative disorder. The method includes: treating a subject, e.g., a patient or an animal, with a protocol under evaluation (e.g., treating a subject with one or more of: chemotherapy, radiation, and/or a compound identified using the methods described herein); and evaluating the expression of a 32144 nucleic acid or polypeptide before and after treatment. A change, e.g., a decrease or increase, in the level of a 32144 nucleic acid (e.g., mRNA) or polypeptide after treatment, relative to the level of expression before treatment, is indicative of the efficacy of the treatment of the disorder. The level of 32144 nucleic acid or polypeptide expression can be detected by any method described herein.

In a preferred embodiment, the evaluating step includes obtaining a sample (e.g., a tissue sample, e.g., a biopsy, or a fluid sample) from the subject, before and after treatment and comparing the level of expressing of a 32144 nucleic acid (e.g., mRNA) or polypeptide before and after treatment.

The invention provides methods for evaluating the efficacy of a therapeutic or prophylactic agent (e.g., an anti-neoplastic agent). The method includes: contacting a sample with an agent (e.g., a compound identified using the methods described herein, a cytotoxic agent) and, evaluating the expression of 32144 nucleic acid or polypeptide in the sample before and after the contacting step. A change, e.g., a decrease or increase, in the level of 32144 nucleic acid (e.g., mRNA) or polypeptide in the sample obtained after the contacting step, relative to the level of expression in the sample before the contacting step, is indicative of the efficacy of the agent. The level of 32144 nucleic acid or polypeptide expression can be detected by any method described herein. In a preferred embodiment, the sample includes cells obtained from a cancerous tissue or a lung, colon, or ovary tissue. In another embodiment, the sample includes cells obtained from a neural tissue or blood cells, e.g., white blood cells.

Other features and advantages of the invention will be apparent from the following detailed description, and from the claims.

### Brief Description of the Drawings

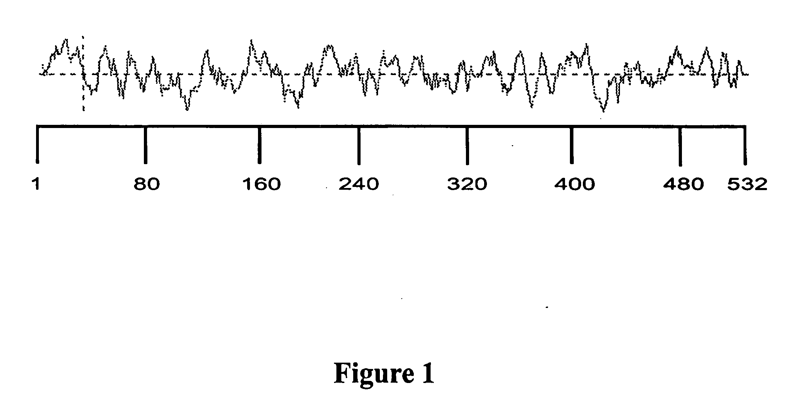

*Figure 1* depicts a hydropathy plot of human 32144. Relative hydrophobic residues are shown above the dashed horizontal line, and relative hydrophilic residues are below the dashed horizontal line. Numbers corresponding to positions in the amino acid sequence of human 32144 are indicated. Polypeptides of the invention include fragments which include: all or part of a hydrophobic sequence, i.e., a sequence above the dashed line, e.g., the sequence from about amino acid 157 to 182, from about 388 to 414, and from about 471 to 491 of SEQ ID NO:2; all or part of a hydrophilic sequence, i.e., a sequence below the dashed line, e.g., the sequence of from about amino acid 104 to 120, from about 183 to 201, and from about 415 to 438 of SEQ ID NO:2.

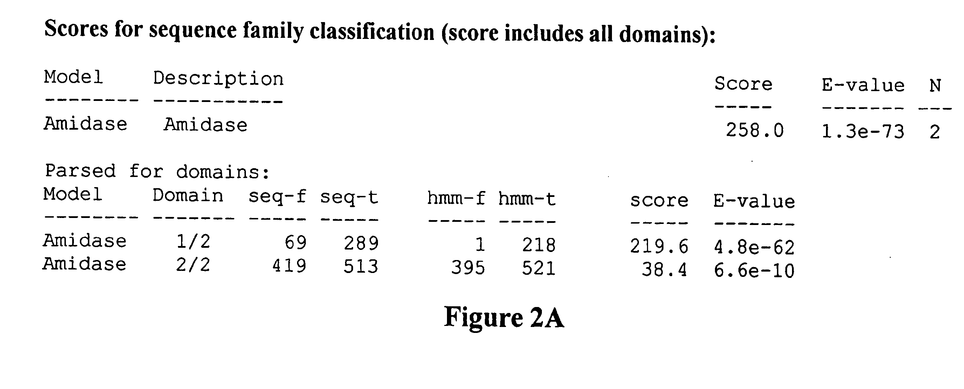

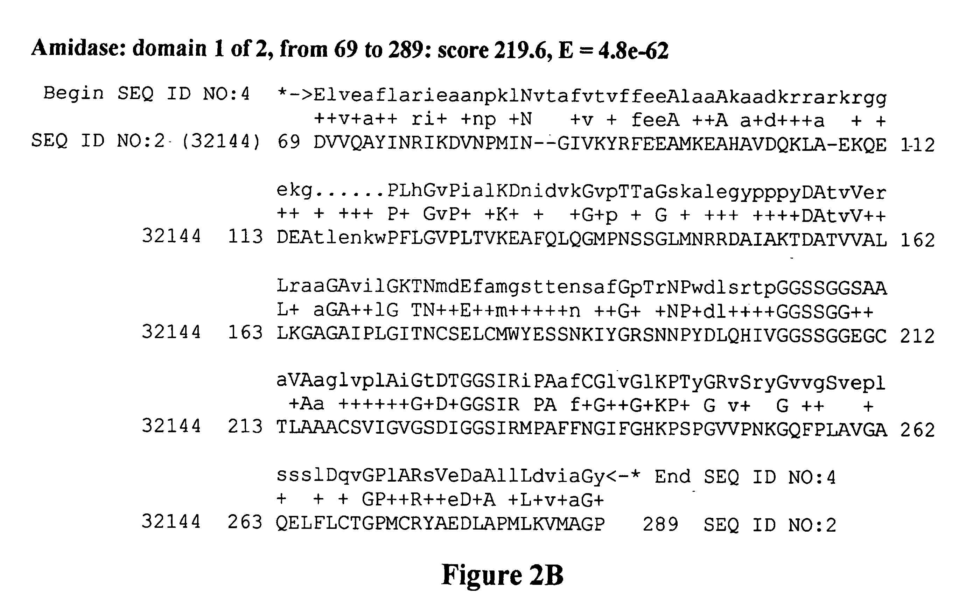

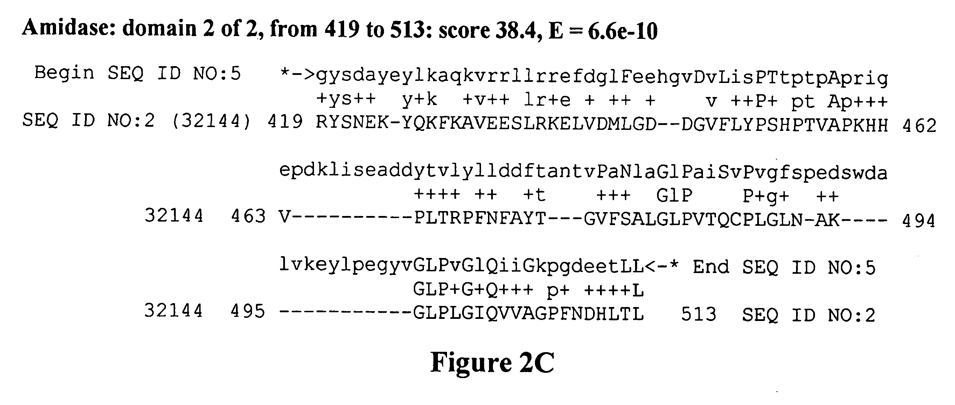

*Figure 2A*-C depicts alignments of portions of the fatty acid amide hydrolase domain of human 32144 with consensus amino acid sequences derived from a hidden Markov model (HMM) from PFAM. The two distinct and non-overlapping consensus amino acid sequences correspond to portions of the PFAM amidase domain, PF01425. Figure 2A describes the scores for the two individual alignments, as well as the combined score for the two alignments. Figure 2B depicts the first of the two alignments. The upper sequence is the consensus amino acid sequence (SEQ ID NO:4) of an N-terminal portion of the amidase domain, while the lower amino acid sequence corresponds to amino acids 69 to 289 of SEQ ID NO:2. Figure 2C depicts the second of the two alignments. The upper sequence is the consensus amino acid sequence (SEQ ID NO:5) of a C-terminal portion of the amidase domain, while the lower amino acid sequence corresponds to amino acids 419 to 513 of SEQ ID NO:2.

### Detailed Description

The human 32144 sequence (see SEQ ID NO:1, as recited in Example 1), which is approximately 2007 nucleotides long including untranslated regions, contains a predicted methionine-initiated coding sequence of about 1599 nucleotides, including the termination codon. The coding sequence encodes a 532 amino acid protein (see SEQ ID NO:2, as recited in Example 1).

Human 32144 contains the following regions or other structural features:
an amidase domain (PFAM Accession Number PF01425) located at about amino acid residues 69 to 289 and 419 to 513 of SEQ ID NO:2;
an amidase signature motif (PS00571) located at about amino acid residues 204 to 235 of SEQ ID NO:2;
a transmembrane domain located at about amino acid residues 11 to 33 of SEQ ID NO:2;
eight predicted Protein Kinase C phosphorylation sites (PS00005) located at about amino acid residues 6 to 8, and 40 to 42, 129 to 131, 186 to 188, 230 to 232, 329 to 331, 365 to 367, and 434 to 436 of SEQ ID NO:2;
three predicted Casein Kinase II phosphorylation sites (PS00006) located at about amino acid residues 129 to 132, 207 to 210, and 320 to 323 of SEQ ID NO:2;
eleven predicted N-myristoylation sites (PS00008) located at about amino acid residues 53 to 58, 125 to 130, 138 to 143, 172 to 177, 204 to 209, 211 to 216, 224 to 229, 248 to 253, 475 to 480, 481 to 486, and 495 to 500 of SEQ ID NO:2;
two predicted N-glycosylation sites (PS00001) at about amino acids 141 to 144 and 175 to 178 of SEQ ID NO:2; and
one predicted microbodies C-terminal targeting signal (PS00342) at about amino acid 530 to 532 of SEQ ID NO:2.

For general information regarding PFAM identifiers, PS prefix and PF prefix domain identification numbers, refer to Sonnhammer et al. (1997) Protein 28:405-420 and http://www.psc.edu/general/ software/packages/pfam/pfam.html.

The 32144 protein contains a significant number of structural characteristics in common with members of the amidase family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., rat or mouse proteins. Members of a family can also have common functional characteristics.

An amidase family of proteins, also referred to as fatty acid amidases hydrolases (FAAH), is characterized by the ability to hydrolyze fatty acid amides, e.g., neuromodulatory fatty acid amides, such as oleamide, anandamide and myristic amide. Representative amidases include fatty acid amide hydrolases (FAAH) from human and mouse (Giang, D.K. et al. (1997) Proc. Natl. Acad. Sci. 94: 2238-2242). Typically, amidases possess substrate specificity based on chain length and degree of saturation of fatty acid amides. Fatty acid amides, e.g., oleamide and ananadmide, are known to have sleep-inducing and analgesic properties, as well as the ability to regulate cellular proliferation. This family of proteins typically contains a highly conserved region rich in glycine, serine and alanine residues. Fatty acid amide hydrolases have been described in Ueda et al. (2000), supra, the contents of which are incorporated herein by reference.

A 32144 polypeptide can include at least one "amidase domain" or "fatty acid amid hydrolase domain", which contains one and preferably two "amidase subdomains" or regions homologous with an "amidase domain".

As used herein, the term "amidase subdomain" or "first amidase subdomain" includes an amino acid sequence of about 100 to 500 amino acid residues in length and having a bit score for the alignment of the sequence to the amidase domain (HMM) of at least 100. Preferably, an amidase domain includes at least about 150 to 450 amino acids, more preferably about 200 to 300 amino acid residues, or about 220 amino acids and has a bit score for the alignment of the sequence to the amidase domain (HMM) of at least 150, preferably 200 or greater: The amidase domain (HMM) has been assigned the PFAM Accession Number PF01425 (http://genome.wustl.edu/Pfam/html). An alignment of the first amidase domain (amino acids 69 to 289 of SEQ ID NO:2) of human 32144 with a consensus amino acid sequence derived from a hidden Markov model is depicted in Figure 2B.

The term "amidase subdomain" or "second amidase subdomain" includes an amino acid sequence of about 40 to 300 in length and having a bit score for the alignment of the sequence to the amidase domain (HMM) of at least 10. Preferably, an amidase domain includes at least about 60 to 200 amino acids, more preferably about 80 to 100 amino acid residues, or about 94 amino acids and has a bit score for the alignment of the sequence to the amidase domain (HMM) of at least 20, preferably 30 or greater. The amidase domain (HMM) has been assigned the PFAM Accession Number PF01425 (http://genome.wustl.edu/Pfam/html). An alignment of the second amidase subdomain (amino acid residues 419 to 513) of human 32144 with a consensus amino acid sequence derived from a hidden Markov model is depicted in Figure 3C.

In a preferred embodiment, a 32144 polypeptide or protein has at least one "amidase subdomain" or a region that includes at least the size ranges described above and has at least about 60%, 70% 80% 90% 95%, 99%, or 100% homology with an "amidase domain," e.g., the amidase subdomain of human 32144 (e.g., residues 69 to 289 or 419 to 513 of SEQ ID NO:2).

To identify the presence of an "amidase" or "fatty acid amide hydrolase" domain in a 32144 protein sequence, and make the determination that a polypeptide or protein of interest has a particular profile, the amino acid sequence of the protein can be searched against the PFAM database ofHMMs (e.g., the Pfam database, release 2.1) using the default parameters (http://www.sanger.ac.uk/Software/Pfam/HNIM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the PFAM database can be found in Sonhammer et al. (1997) Proteins 28(3):405-420 and a detailed description of HMMs can be found, for example, in Gribskov et al.(1990) Meth. Enzymol. 183:146-159; Gribskov et al. (1987) Proc. Natl. Acad Sci. USA 84:4355-4358; Krogh et al.(1994) J. Mol. Biol. 235:1501-1531; and Stultz et al.(1993) Protein Sci. 2:305-314, the contents of which are incorporated herein by reference. A search was performed against the HMM database resulting in the identification of a "amidase" domain in the amino acid sequence of human 32144, which includes two amidase subdomains located at about amino acid residues 69 to 289 and 419 to 513 of SEQ ID NO:2 (see Figure 2).

In one embodiment, a 32144 protein includes at least one amidase signature motif. As used herein, an "amidase signature motif" includes a sequence of at least nineteen amino acid residues defined by the sequence: G-[G/A]-S-[G/S]-[G/S]-G-X-[G/S/A]-[G/S/A/V/Y]-X-[G/A]-X-[D/E]-X-[G/A]-X-S-[L/l/V/M]-R-X-P-[G/S/A/C] (SEQ ID NO:6). An amidase signature motif, as defined, can be involved in the enzymatic hydrolysis of a fatty acid amide. More preferably, an amidase signature motif includes 25, 29, or even more preferably 32 amino acid residues. Amidase signature motifs have been described in, e.g., Mayaux et al. (1990), J Bacteriology 172:6764-73, the contents of which are incorporated herein by reference. Human 32144 contains a sequence (about amino acid residues 204-235 of SEQ ID NO:2) which matches the sequence of an amide signature motif at 18/19 of the conserved positions. The single discrepancy occurs at position 9 ([G/S/A/V/Y]) of the amidase signature sequence, where there is a conservative cystein substitution (located at about amino acid residue 212 of SEQ ID NO:2) observed in human 32144.

In a preferred embodiment, a 32144 polypeptide or protein has at least one amidase signature motif, or a region which includes at least 19, 25, 29, or even 32 amino acid residues and has at least 70%, 80%, 90%, or 100% homology with an "amidase signature motif" or the variant amidase signature motif observed in human 32225, e.g., about amino acid residues 204 to 235 of SEQ ID NO: 2.

A 32144 molecule can further include a transmembrane region. As used herein, the term "transmembrane domain" includes an amino acid sequence of at least about 14 amino acid residues in length that spans a phospholipid membrane. More preferably, a transmembrane domain includes at least about 14, 16, 18, 20, 22, or 24 amino acid residues and spans a phospholipid membrane. Transmembrane domains are rich in hydrophobic residues, and typically have an α-helical structure. In a preferred embodiment, at least 50%, 60%, 70%, 80%, 90%, 95% or more of the amino acids of a transmembrane domain are hydrophobic, e.g., leucines, valines, alanines, phenylalanines, methionines, isoleucines, tyrosines, or tryptophans. Transmembrane domains are described in, for example, Zagotta W.N. et al., (1996) Annual Rev. Neuronsci. 19:235-63.

In a preferred embodiment, a 32144 polypeptide or protein has at least one transmembrane domain or a region which includes at least 18, 19, or 20 amino acid residues and has at least about 60%, 70%, 80%, 90%, 95%, 99%, or 100% homology with a "transmembrane domain," e.g., at least one transmembrane domain of human 32144 (e.g., from about amino acid residues 11 to 33 of SEQ ID NO:2). In one embodiment, the transmembrane domain of a 32144 molecule is able to interact with transmembrane domains of other molecules, e.g. other 32144 molecules, such that the 32144 forms an oligomer, e.g., a homooligomer. The self-association of fatty acid amide hydrolases via N-termial transmembrane domains has been described in Ueda et al. (2000), supra.

A 32144 family member can include at least one, and preferably two amidase subdomains. Furthermore, a 32144 family member can include at least one amidase signature motif; at least one transmembrane domain; at least one, two, three, four, five, six, seven, and preferably eight predicted protein kinase C phosphorylation sites (PS00005); at least one, two, and preferably three predicted casein kinase II phosphorylation sites (PS00006); at least one, two, three, four, five, six, seven, eight, nine, ten, and preferably eleven predicted N-myristylation sites (PS00008); at least one, and preferably two predicted N-glycosylation sites (PS00001); and at least one predicted Microbodies C-terminal targeting signal (PS00342).

As the 32144 polypeptides of the invention may modulate 32144-mediated activities, they may be useful as of for developing novel diagnostic and therapeutic agents for 32144-mediated or related disorders, as described below.

As used herein, a "32144 activity", "biological activity of 32144" or "functional activity of 32144", refers to an activity exerted by a 32144 protein, polypeptide or nucleic acid molecule. For example, a 32144 activity can be an activity exerted by 32144 in a physiological milieu on, e.g.; a 32144-responsive cell or on a 32144 substrate, e.g., a protein substrate. A 32144 activity can be determined *in vivo* or *in vitro.* In one embodiment, a 32144 activity is a direct activity, such as an association with a 32144 target molecule. A "target molecule" or "binding partner" is a molecule with which a 32144 protein binds or interacts in nature. In an exemplary embodiment, 32144 is an enzyme that hydrolyses fatty acid amides, e.g., anandamide or ethanolamides of oleic (e.g., oleamide), linoleic, or palmitic acids.

A 32144 activity can also be an indirect activity, e.g., a cellular signaling activity mediated by interaction of the 32144 protein with a 32144 receptor. The features of the 32144 molecules of the present invention can provide similar biological activities as fatty acid amide hydrolase family members. For example, the 32144 proteins of the present invention can have one or more of the following activities: (1) bind and catabolize fatty acid amides; (2) regulate neuronal signaling; (3) regulate ion channel function, e.g., 5-HT₃ ion channel function; (4) regulate cannabinoid receptor signaling; (5) regulate seratonin signaling, e.g., 5-HT₂ response to seratonin; (6) regulate gap junction activity; (7) regulate pain reception; (8) regulate development; (9) regulate cellular proliferation and/or migration; (10) regulate focal adhesion kinase activity; or (11) regulate the induction of sleep.

Thus, the 32144 molecules can act as novel diagnostic targets and therapeutic agents for controlling cellular proliferation and/or differentiation disorders including those of colon, lung, and ovarian origin.

As used herein, the terms "cancer", "hyperproliferative" and "neoplastic" refer to cells having the capacity for autonomous growth. Examples of such cells include cells having an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

Examples of cellular proliferative and/or differentiative disorders of the colon include, but are not limited to, non-neoplastic polyps, adenomas, familial syndromes, colorectal carcinogenesis, colorectal carcinoma, and carcinoid tumors.

Examples of cellular proliferative and/or differentiative disorders of the lung include, but are not limited to, bronchogenic carcinoma, including paraneoplastic syndromes, bronchioloalveolar carcinoma, neuroendocrine tumors, such as bronchial carcinoid, miscellaneous tumors, and metastatic tumors; pathologies of the pleura, including inflammatory pleural effusions, noninflammatory pleural effusions, pneumothorax, and pleural tumors, including solitary fibrous tumors (pleural fibroma) and malignant mesothelioma.

The 32144 protein, fragments thereof, and derivatives and other variants of the sequence in SEQ ID NO:2 thereof are collectively referred to as "polypeptides or proteins of the invention" or "32144 polypeptides or proteins". Nucleic acid molecules encoding such polypeptides or proteins are collectively referred to as "nucleic acids of the invention" or "32144 nucleic acids." 32144 molecules refer to 32144 nucleic acids, polypeptides, and antibodies.

As used herein, the term "nucleic acid molecule" includes DNA molecules (e.g., a cDNA or genomic DNA), RNA molecules (e.g., an mRNA) and analogs of the DNA or RNA. A DNA or RNA analog can be synthesized from nucleotide analogs. The nucleic acid molecule can be single-stranded or double-stranded, but preferably is double-stranded DNA.

The term "isolated nucleic acid molecule" or "purified nucleic acid molecule" includes nucleic acid molecules that are separated from other nucleic acid molecules present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

Preferably, an isolated nucleic acid molecule that hybridizes under a stringency condition described herein to the sequence of SEQ ID NO:1 or SEQ ID NO:3, corresponds to a naturally-occurring nucleic acid molecule.

As used herein, a "naturally-occurring" nucleic acid molecule refers to an RNA or DNA molecule having a nucleotide sequence that occurs in nature. For example a naturally occurring nucleic acid molecule can encode a natural protein.

As used herein, the terms "gene" and "recombinant gene" refer to nucleic acid molecules which include at least an open reading frame encoding a 32144 protein. The gene can optionally further include non-coding sequences, e.g., regulatory sequences and introns. Preferably, a gene encodes a mammalian 32144 protein or derivative thereof.

An "isolated" or "purified" polypeptide or protein is substantially free of cellular material or other contaminating proteins from the cell or tissue source from which the protein is derived, or substantially free from chemical precursors or other chemicals when chemically synthesized. "Substantially free" means that a preparation of 32144 protein is at least 10% pure. In a preferred embodiment, the preparation of 32144 protein has less than about 30%, 20%, 10% and more preferably 5% (by dry weight), of non-32144 protein (also referred to herein as a "contaminating protein"), or of chemical precursors or non-32144 chemicals. When the 32144 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 32144 without abolishing or substantially altering a 32144 activity. Preferably the alteration does not substantially alter the 32144 activity, e.g., the activity is at least 20%, 40%, 60%, 70% or 80% of wild-type. An "essential" amino acid residue is a residue that, when altered from the wild-type sequence of 32144, results in abolishing a 32144 activity such that less than 20% of the wild-type activity is present. For example, conserved amino acid residues in 32144 are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 32144 protein is preferably replaced with another amino acid residue from the same side chain family. Alternatively, in another embodiment, mutations can be introduced randomly along all or part of a 32144 coding sequence, such as by saturation mutagenesis, and the resultant mutants can be screened for 32144 biological activity to identify mutants that retain activity. Following mutagenesis of SEQ ID NO:1 or SEQ ID NO: 3, the encoded protein can be expressed recombinantly and the activity of the protein can be determined.

As used herein, a "biologically active portion" of a 32144 protein includes a fragment of a 32144 protein which participates in an interaction, e.g., an intramolecular or an inter-molecular interaction. An inter-molecular interaction can be a specific binding interaction or an enzymatic interaction (e.g., the interaction can be transient and a covalent bond is formed or broken). An inter-molecular interaction can be between a 32144 molecule and a non-32144 molecule or between a first 32144 molecule and a second 32144 molecule (e.g., a dimerization interaction). Biologically active portions of a 32144 protein include peptides comprising amino acid sequences sufficiently homologous to or derived from the amino acid sequence of the 32144 protein, e.g., the amino acid sequence shown in SEQ ID NO:2, which include less amino acids than the full length 32144 proteins, and exhibit at least one activity of a 32144 protein. Typically, biologically active portions comprise a domain or motif with at least one activity of the 32144 protein, e.g., the hydrolysis of fatty acid amides. A biologically active portion of a 32144 protein can be a polypeptide which is, for example, 10, 25, 50, 100, 200 or more amino acids in length. Biologically active portions of a 32144 protein can be used as targets for developing agents which modulate a 32144-mediated activity, e.g., modulation of neuronal signaling, sleep induction, pain perception, or cellular proliferation and/or differentiation.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CNV matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN-program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to 32144 nucleic acid molecules of the invention. BLAST protein searches can be performed with the XBLAST program, score = 50, wordlength = 3 to obtain amino acid sequences homologous to 32144 protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov.

Particularly preferred 32144 polypeptides have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO:1 or 3 are termed substantially identical.

"Misexpression or aberrant expression", as used herein, refers to a non-wildtype pattern of gene expression at the RNA or protein level. It includes: expression at non-wild type levels, i.e., over- or under-expression; a pattern of expression that differs from wild type in terms of the time or stage at which the gene is expressed, e.g., increased or decreased expression (as compared with wild type) at a predetermined developmental period or stage; a pattern of expression that differs from wild type in terms of altered, e.g., increased or decreased, expression (as compared with wild type) in a predetermined cell type or tissue type; a pattern of expression that differs from wild type in terms of the splicing size, translated amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, refers to human and non-human animals. The term "non-human animals" of the invention includes all vertebrates, e.g., mammals, such as non-human primates (particularly higher primates), sheep, dog, rodent (e.g., mouse or rat), guinea pig, goat, pig, cat, rabbits, cow, and non-mammals, such as chickens, amphibians, reptiles, etc. In a preferred embodiment, the subject is a human. In another embodiment, the subject is an experimental animal or animal suitable as a disease model.

A "purified preparation of cells", as used herein, refers to an in vitro preparation of cells. In the case cells from multicellular organisms (e.g., plants and animals), a purified preparation of cells is a subset of cells obtained from the organism, not the entire intact organism. In the case of unicellular microorganisms (e.g., cultured cells and microbial cells), it consists of a preparation of at least 10% and more preferably 50% of the subject cells.

Various aspects of the invention are described in further detail below.

### Isolated Nucleic Acid Molecules

Disclosed herein is an isolated or purified, nucleic acid molecule that encodes a 32144 polypeptide described herein, e.g., a full-length 32144 protein or a fragment thereof, e.g., a biologically active portion of 32144 protein. Also disclosed is a nucleic acid fragment suitable for use as a hybridization probe, which can be used, e.g., to identify a nucleic acid molecule encoding a polypeptide of the invention, 32144 mRNA, and fragments suitable for use as primers, e.g., PCR primers for the amplification or mutation of nucleic acid molecules.

In one aspect a 32144 isolated nucleic acid molecule includes the nucleotide sequence shown in SEQ ID NO: 1, or a portion of any of these nucleotide sequences. In one embodiment, the nucleic acid molecule includes sequences encoding the human 32144 protein (i.e., "the coding region" of SEQ ID NO:1, as shown in SEQ ID NO:3), as well as 5' untranslated sequences. Alternatively, the nucleic acid molecule can include only the coding region of SEQ ID NO:1 (e.g., SEQ ID NO: 3) and, e.g., no flanking sequences which normally accompany the subject sequence. In another embodiment, the nucleic acid molecule encodes a sequence corresponding to a fragment of the protein from about amino acid residues 34 to 532 of SEQ ID NO:2.

In another aspect, a 32144 isolated nucleic acid molecule includes a nucleic acid molecule which is a complement of the nucleotide sequence shown in SEQ ID NO:1 or SEQ ID NO:3, or a portion of any of these nucleotide sequences. Alternatively, the nucleic acid molecule is sufficiently complementary to the nucleotide sequence shown in SEQ ID NO:1 or SEQ ID NO:3, such that it can hybridize (e.g., under a stringency condition described herein) to the nucleotide sequence shown in SEQ ID NO:1 or 3, thereby forming a stable duplex.

In one aspect, an isolated nucleic acid molecule includes a nucleotide sequence which is at least about: 60%, 65%, 70%, 75%, 80%, 85%, 90%, 91%, 92%, 93%, 94%, 95%, 96%, 97%, 98%, 99%, or more homologous to the entire length of the nucleotide sequence shown in SEQ ID NO:1 or SEQ ID NO:3, or a portion, preferably of the same length, of any of these nucleotide sequences.

### 32144 Nucleic Acid Fragments

A 32144 nucleic acid molecule can include only a portion of the nucleic acid sequence of SEQ ID NO:1 or 3. For example, such a nucleic acid molecule can include a fragment which can be used as a probe or primer or a fragment encoding a portion of a 32144 protein, e.g., an immunogenic or biologically active portion of a 32144 protein. A fragment can comprise those nucleotides of SEQ ID NO: 1, which encode a fatty acid amide hydrolase domain of human 32144. The nucleotide sequence determined from the cloning of the 32144 gene allows for the generation of probes and primers designed for use in identifying and/or cloning other 32144 family members, or fragments thereof, as well as 32144 homologues, or fragments thereof, from other species.

In another aspect, a nucleic acid includes a nucleotide sequence that includes part, or all, of the coding region and extends into either (or both) the 5' or 3' noncoding region. Other aspects include a fragment which includes a nucleotide sequence encoding an amino acid fragment described herein. Nucleic acid fragments can encode a specific domain or site described herein or fragments thereof, particularly fragments thereof which are at least 50, 100, 150, 200, 250, 300, 350, 400, 425, 450, 475, 500 or more amino acids in length. Preferably, the nucleic acid fragments encode a specific domain or fragment thereof, wherein the domain or fragment is at least 125, or more preferably 140, 145, or even 150 amino acids in length. Fragments also include nucleic acid sequences corresponding to specific amino acid sequences described above or fragments thereof Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 32144 nucleic acid fragment can include a sequence corresponding to a fatty acid amide hydrolase domain, an amidase signature motif, or a transmembrane domain.

32144 probes and primers are disclosed. Typically a probe/primer is an isolated or purified oligonucleotide. The oligonucleotide typically includes a region of nucleotide sequence that hybridizes under a stringency condition described herein to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO: 1 or SEQ ID NO:3. Preferably, an oligonucleotide is less than about 200, 150, 120, or 100 nucleotides in length.

In one embodiment, the probe or primer is attached to a solid support, e.g., a solid support described herein.

One exemplary kit of primers includes a forward primer that anneals to the coding strand and a reverse primer that anneals to the non-coding strand. The forward primer can anneal to the start codon, e.g., the nucleic acid sequence encoding amino acid residue 1 of SEQ ID NO:2. The reverse primer can anneal to the ultimate codon, e.g., the codon immediately before the stop codon, e.g., the codon encoding amino acid residue 532 of SEQ ID NO:2. In a preferred aspect, the annealing temperatures of the forward and reverse primers differ by no more than 5, 4, 3, or 2°C.

Preferably, the nucleic acid is a probe which is at least 10, 12, 15, 18, 20 and less than 200, more preferably less than 100, or less than 50, nucleotides in length. It should be identical, or differ by 1, or 2, or less than 5 or 10 nucleotides, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: a fatty acid amide hydrolase domain or a fragment thereof of human 32144, e.g., about amino acid residues 69 to 513, 69 to 289, or 419 to 513 of SEQ ID NO:2; a amidase signature motif of human 32144, e.g., about amino acid residues 204 to 235 of SEQ ID NO:2; or a transmembrane domain of human 32144, e.g., about amino acids 11 to 33 of SEQ ID NO:2.

In another aspect a set of primers is disclosed, e.g., primers suitable for use in a PCR, which can be used to amplify a selected region of a 32144 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variants. For example, primers suitable for amplifying all or a portion of any of the following regions are provided: a fatty acid amide hydrolase domain or fragment thereof, e.g., from about amino acid 69 to 513, 69 to 289, or 419 to 513 of SEQ ID NO:2; an amidase signature motif, e.g., about amino acid residues 204 to 235 of SEQ ID NO:2; or an N-terminal fragment, e.g., about amino acid residues 1 to 68 or 33 to 68 of SEQ ID NO:2.

A nucleic acid fragment can encode an epitope bearing region of a polypeptide described herein.

A nucleic acid fragment encoding a "biologically active portion of a 32144 polypeptide" can be prepared by isolating a portion of the nucleotide sequence of SEQ ID NO:1 or 3, which encodes a polypeptide having a 32144 biological activity (e.g., the biological activities of the 32144 proteins are described herein), expressing the encoded portion of the 32144 protein (e.g., by recombinant expression *in vitro)* and assessing the activity of the encoded portion of the 32144 protein. For example, a nucleic acid fragment encoding a biologically active portion of 32144 includes a fatty acid amide hydrolase domain, e.g., about amino acid residues 69 to 513 of SEQ ID NO:2, or a transmembrane domain, e.g., about amino acid residues 11 to 33 of SEQ ID NO:2. A nucleic acid fragment encoding a biologically active portion of a 32144 polypeptide, may comprise a nucleotide sequence which is greater than 300, 400, 500, 550 or more nucleotides in length.

Preferably, a nucleic acid includes a nucleotide sequence which is about 300, 400, 500, 600, 700, 800, 900, 1000, 1100, 1200, 1300, 1400, 1500, 1600, 1700, 1800, 1900, 2000 or more nucleotides in length and hybridizes under a stringency condition described herein to a nucleic acid molecule of SEQ ID NO:1, or SEQ ID NO:3.

Preferably, a nucleic acid fragment differs by at least 1, 2, 3, 10, 20, or more nucleotides from the sequence of Genbank accession number AA902127, AF086253, AI248721, AI457475, AI811051, AL158750, BE217829, BE504565, BE549648, BE671210, G37418, or Z83745, or SEQ ID NO:3014 of WO 01/02568. Differences can include differing in length or sequence identity. For example, a nucleic acid fragment can: include one or more nucleotides from SEQ ID NO: 1 or SEQ ID NO:3 located outside the region of nucleotides 66 to 533, 999 to 1121, or 1415 to 1985; not include all of the nucleotides of AA902127 or AI457475, e.g., can be one or more nucleotides shorter (at one or both ends) than the sequence of AI457475; or can differ by one or more nucleotides in the region of overlap.

### 32144 Nucleic Acid Variants

Further disclosed are nucleic acid molecules that differ from the nucleotide sequence shown in SEQ ID NO:1 or SEQ ID NO:3. Such differences can be due to degeneracy of the genetic code (and result in a nucleic acid which encodes the same 32144 proteins as those encoded by the nucleotide sequence disclosed herein. Alternatively, the isolated nucleic acid molecule has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. The encoded protein can differ by no more than 5, 4, 3, 2, or 1 amino acid. "Looped" out sequences from deletions or insertions, or mismatches, are considered differences.

Nucleic acids can be chosen for having codons, which are preferred, or non-preferred, for a particular expression system. E.g., the nucleic acid can be one in which at least one codon, at preferably at least 10%, or 20% of the codons has been altered such that the sequence is optimized for expression in *E*. *coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

Preferably, the nucleic acid differs from that of SEQ ID NO: 1 or 3, e.g., as follows: by at least one but less than 10, 20, 30, or 40 nucleotides; at least one but less than 1 %, 5%, 10% or 20% of the nucleotides in the subject nucleic acid. The nucleic acid can differ by no more than 5, 4, 3, 2, or 1 nucleotide. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under a stringency condition described herein, to the nucleotide sequence shown in SEQ ID NO 2 or a fragment of the sequence. Nucleic acid molecules corresponding to orthologs, homologs, and allelic variants of the 32144 cDNAs of the invention can further be isolated by mapping to the same chromosome or locus as the 32144 gene.

Preferred variants include those that are correlated with the ability to enzymatically hydrolyze fatty acid amides, e.g., anandamide or ethanolamides of oleic (e.g., oleamide), linoleic, or palmitic acids.

Allelic variants of 32144, e.g., human 32144, include both functional and non-functional proteins. Functional allelic variants are naturally occurring amino acid sequence variants of the 32144 protein within a population that maintain the ability to bind and catabolize fatty acid amides, e.g., anandamide or ethanolamides of oleic (e.g., oleamide), linoleic, or palmitic acids. Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 32144, e.g., human 32144, protein within a population that do not have the ability to bind and catabolize fatty acid amides, e.g., anandamide or ethanolamides of oleic (e.g., oleamide), linoleic, or palmitic acids. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 32144 family members and, thus, which have a nucleotide sequence which differs from the 32144 sequences of SEQ ID NO:1 or SEQ ID NO:3 are also disclosed.

### Antisense Nucleic Acid Molecules, Ribozymes and Modified 32144 Nucleic Acid Molecules

Further disclosed herein is an isolated nucleic acid molecule which is antisense to 32144. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 32144 coding strand, or to only a portion thereof (e.g., the coding region of human 32144 corresponding to SEQ ID NO:3). In another aspect, the antisense nucleic acid molecule is antisense to a "noncoding region" of the coding strand of a nucleotide sequence encoding 32144 (e.g., the 5' and 3' untranslated regions).

An antisense nucleic acid can be designed such that it is complementary to the entire coding region of 32144 mRNA, but more preferably is an oligonucleotide which is antisense to only a portion of the coding or noncoding region of 32144 mRNA. For example, the antisense oligonucleotide can be complementary to the region surrounding the translation start site of 32144 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules are typically administered to a subject (e.g., by direct injection at a tissue site), or generated *in situ* such that they hybridize with or bind to cellular mRNA and/or genomic DNA encoding a 32144 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another aspect, the antisense nucleic acid molecule is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330)*.*

In still another aspect, the antisense nucleic acid is a ribozyme. A ribozyme having specificity for a 32144-encoding nucleic acid can include one or more sequences complementary to the nucleotide sequence of a 32144 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) Nature 334:585-591). For example, a derivative of a *Tetrahymena* L-19 IVS RNA can be constructed in which the nucleotide sequence of the active site is complementary to the nucleotide sequence to be cleaved in a 32144-encoding mRNA. See, e.g., Cech et al. U.S. Patent No. 4,987,071; and Cech et al. U.S. Patent No. 5,116,742. Alternatively, 32144 mRNA can be used to select a catalytic RNA having a specific ribonuclease activity from a pool of RNA molecules. See, e.g., Bartel, D. and Szostak, J.W. (1993) Science 261:1411-1418.

32144 gene expression can be inhibited by targeting nucleotide sequences complementary to the regulatory region of the 32144 (e.g., the 32144 promoter and/or enhancers) to form triple helical structures that prevent transcription of the 32144 gene in target cells. See generally, Helene, C. (1991) Anticancer Drug Des. 6:569-84; Helene, C. i (1992) Ann. N.Y. Acad Sci. 660:27-36; and Maher, L.J: (1992) Bioassays 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Also disclosed are detectably labeled oligonucleotide primer and probe molecules. Typically, such labels are chemiluminescent, fluorescent, radioactive, or colorimetric.

A 32144 nucleic acid molecule can be modified at the base moiety, sugar moiety or phosphate backbone to improve, e.g., the stability, hybridization, or solubility of the molecule. For non-limiting examples of synthetic oligonucleotides with modifications see Toulmé (2001) Nature Biotech. 19:17 and Faria et al. (2001) Nature Biotech. 19:40-44. Such phosphoramidite oligonucleotides can be effective antisense agents.

For example, the deoxyribose phosphate backbone of the nucleic acid molecules can be modified to generate peptide nucleic acids (see Hyrup B. et al. (1996) Bioorganic & Medicinal Chemistry 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup **B**. *et al.* (1996) *supra* and Perry-O'Keefe el al. Proc. Natl. Acad. Sci. 93; 14670-675.

PNAs of 32144 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 32144 nucleic acid molecules can also be used in the analysis of single base pair mutations in a gene, (e.g., by PNA-directed PCR clamping); as 'artificial restriction enzymes' when used in combination with other enzymes, (e.g., S 1 nucleases (Hyrup B. *et al.* (1996) *supra*))*;* or as probes or primers for DNA sequencing or hybridization (Hyrup B*. et al.* (1996) *supra;* Perry-O'Keefe *supra).*

Alternatively, the oligonucleotide may include other appended groups such as peptides (e.g., for targeting host cell receptors *in vivo*), or agents facilitating transport across the cell membrane (see, e.g., Letsinger et al. (1989) Proc. Natl. Acad Sci. USA 86:6553-6556; Lemaitre et al. (1987) Proc. Natl. Acad. Sci. USA 84:648-652; PCT Publication No. WO88/09810) or the blood-brain barrier (see, e.g., PCT Publication No. WO89/10134). In addition, oligonucleotides can be modified with hybridization-triggered cleavage agents (see, e.g., Krol et al. (1988) Bio-Techniques 6:958-976) or intercalating agents. (see, e.g., Zon (1988) Pharm. Res. 5:539-549). To this end, the oligonucleotide may be conjugated to another molecule, (e.g., a peptide, hybridization triggered cross-linking agent, transport agent, or hybridization-triggered cleavage agent).

Also disclosed are molecular beacon-oligonucleotide-primer and probe molecules having at least one region which is complementary to a 32144 nucleic acid of the invention, two complementary regions one having a fluorophore and one a quencher such that the molecular beacon is useful for quantitating the presence of the 32144 nucleic acid of the invention in a sample. Molecular beacon nucleic acids are described, for example, in Lizardi et al., U.S. Patent No. 5,854,033; Nazarenko el al., U.S. Patent No. 5,866,336, and Livak et al., U.S. Patent 5,876,930.

### Isolated 32144 Polypeptides

Further disclosed is an isolated 32144 protein, or fragment, e.g., a biologically active portion, for use as immunogens or antigens to raise or test (or more generally to bind) anti-32144 antibodies. 32144 protein can be isolated from cells or tissue sources using standard protein purification techniques. 32144 protein or fragments thereof can be produced by recombinant DNA techniques or synthesized chemically.

Polypeptides include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when expressed the polypeptide is expressed in a native cell, or in systems which result in the alteration or omission of post-translational modifications, e.g., glycosylation or cleavage, present when expressed in a native cell.

Preferably, a 32144 polypeptide has one or more of the following characteristics:
(i) it has the ability to bind and catabolize fatty acid amides;
(ii) it has a molecular weight, e.g., a deduced molecular weight, preferably ignoring any contribution of post translational modifications, amino acid composition or other physical characteristic of SEQ ID NO:2;
(iii) it has an overall sequence similarity of at least 50%, preferably at least 60%, more preferably at least 70, 80, 90, or 95%, with a polypeptide a of SEQ ID NO:2;
(iv) it can be found in the lung, liver, colon, breast, ovary, pancreas, kidney, or brain;
(v) it has an amidase domain which is preferably about 70%, 80%, 90% or 95% homologous with amino acid residues about 69 to 513 of SEQ ID NO:2;
(vi) it has an amidase signature motif or a sequence which is about 70%, 80%, 90% or 95% homologous with amino acid residues about 204 to 235 of SEQ ID NO:2;
(vii) it has an N-terminally located transmembrane domain, or a region which is about 70%, 80%, 90% or 95% with amino acid residues about 11 to 33 of SEQ ID NO:2;
(viii) it localizes to cellular membranes;
(ix) it has at least one, two, three, four, five, six, seven, preferably eight predicted Protein kinase C phosphorylation sites (PS00005);
(x) it has at least one, two, preferably three predicted Casein kinase II phosphorylation sites (PS00006);
(xi) it has at least one, two, three, four, five, six, seven, eight, nine, ten, preferably eleven predicted N-myristoylation sites (PS00008);
(xii) it has at least one, preferably two predicted N-glycosylation sites (PS00001); and
(xiii) it has at least one predicted Microbodies C-terminal targeting signal (PS00342).

Preferably the 32144 protein, or fragment thereof, differs from the corresponding sequence in SEQ ID:2. In one aspect it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (If this comparison requires alignment the sequences should be aligned for maximum homology. "Looped" out sequences from deletions or insertions, or mismatches, are considered differences.) The differences are, preferably, differences or changes at a non essential residue or a conservative substitution. Alternatively the differences are not in the fatty acid amide hydrolase domain, e.g., about amino acids 69 to 289 and 4 1 9 to 5 1 3 of SEQ ID NO:2, Alternatively one or more differences are in the fatty acid amide hydrolase domain e.g., about amino acids 69 to 289 and 419 to 513 of SEQ ID NO:2.

Also disclosed is a protein that contain one or more changes in amino acid sequence, e.g., a change in an amino acid residue which is not essential for activity. Such 32144 proteins differ in amino acid sequence from SEQ ID NO:2, yet retain biological activity.

In one aspect the protein includes an amino acid sequence at least about 50%, 55%, 60%, 65%, 70%, 75%, 80%, 85%, 90%, 95%, 98% or more homologous to SEQ ID NO:2.

A 32144 protein or fragment is disclosed which varies from the sequence of SEQ ID NO:2 in regions defined by amino acids about 1 to 10, 34 to 68, 290 to 418, and 514 to 532 by at least one but by less than 15, 10 or 5 amino acid residues in the protein or fragment but which does not differ from SEQ ID NO:2 in regions defined by amino acids about 11 to 33, 69 to 289, and 419 to 513. A 32144 protein or fragment is also disclosed which varies from the sequence of SEQ ID NO:2 in regions defined by amino acids about 11 to 33, 69 to 289, and 419 to 513 by at least one but by less than 15, 10 or 5 amino acid residues in the protein or fragment but which does not differ from SEQ ID NO:2 in regions defined by amino acids about 1 to 10, 34 to 68, 290 to 418, and 514 to 532. (If these comparisons require alignment, the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one Aspect, a biologically active portion of a 32144 protein includes a fatty acid amide hydrolase domain or a transmembrane domain. Moreover, other biologically active portions, in which other regions of the protein are deleted, can be prepared by recombinant techniques and evaluated for one or more of the functional activities of a native 32144 protein.

Preferably, the 32144 protein has an amino acid sequence shown in SEQ ID NO:2. Alternatively, the 32144 protein is substantially identical to SEQ ID NO:2. Alternatively, the 32144 protein is substantially identical to SEQ ID NO:2 and retains the functional activity of the protein of SEQ ID NO:2, as described in detail in the subsections above.

Preferably, a fragment differs by at least 1, 2, 3, 10, 20, or more amino acid residues encoded by a sequence in AA902127, AF086253, AI248721, AI457475, AI811051, AL158750, BE217829, BE504565, BE549648, BE671210, G37418, or Z83745, or SEQ ID NO:3014 of WO 01/02568. Differences can include differing in length or sequence identity. For example, a fragment can: include one or more amino acid residues from SEQ ID NO: 2 outside the region encoded by nucleotides 119 to 533, 999 to 1121, or 1467 to 1717; not include all of the amino acid residues of a sequence in AA902127 or AI457475, e.g., can be one or more amino acid residues shorter (at one or both ends) than a sequence in AA902127 or AI457475; or can differ by one or more amino acid residues in the region of overlap.

### Variants of 32144 Proteins

Further disclosed is a variant of a 32144 polypeptide, e.g., which functions as an agonist (mimetics) or as an antagonist. Variants of the 32144 proteins can be generated by mutagenesis, e.g., discrete point mutation, the insertion or deletion of sequences or the truncation of a 32144 protein. An agonist of the 32144 proteins can retain substantially the same, or a subset, of the biological activities of the naturally occurring form of a 32144 protein. An antagonist of a 32144 protein can inhibit one or more of the activities of the naturally occurring form of the 32144 protein by, for example, competitively modulating a 32144-mediated activity of a 32144 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 32144 protein.

Variants of a 32144 protein can be identified by screening combinatorial libraries of mutants, e.g., truncation mutants, of a 32144 protein for agonist or antagonist activity.

Libraries of fragments e.g., N terminal, C terminal, or internal fragments, of a 32144 protein coding sequence can be used to generate a variegated population of fragments for screening and subsequent selection of variants of a 32144 protein. Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of 32144 proteins. Recursive ensemble mutagenesis (REM), a new technique which enhances the frequency of functional mutants in the libraries, can be used in combination with the screening assays to identify 32144 variants (Arkin and Yourvan (1992) Proc. Natl. Acad Sci. USA 89:7811-7815; Delgrave et al. (1993) Protein Engineering 6:327-331).

Cell based assays can be exploited to analyze a variegated 32144 library. For example, a library of expression vectors can be transfected into a cell line, e.g., a cell line, which ordinarily responds to 32144 in a substrate-dependent manner. The transfected cells are then contacted with 32144 and the effect of the expression of the mutant on signaling by the 32144 substrate can be detected, e.g., by measuring seratonin signaling, ion channel activity, focal adhesion kinase activity, or cellular proliferation or migration. Plasmid DNA can then be recovered from the cells which score for inhibition, or alternatively, potentiation of signaling by the 32144 substrate, and the individual clones further characterized.

Further disclosed is a method of making a 32144 polypeptide, e.g., a peptide having a non-wild type activity, e.g., an antagonist, agonist, or super agonist of a naturally occurring 32144 polypeptide, e.g., a naturally occurring 32144 polypeptide. The method includes: altering the sequence of a 32144 polypeptide, e.g., altering the sequence , e.g., by substitution or deletion of one or more residues of a non-conserved region, a domain or residue disclosed herein, and testing the altered polypeptide for the desired activity.

Also disclosed is a method of making a fragment or analog of a 32144 polypeptide a biological activity of a naturally occurring 32144 polypeptide. The method includes: altering the sequence, e.g., by substitution or deletion of one or more residues, of a 32144 polypeptide, e.g., altering the sequence of a non-conserved region, or a domain or residue described herein, and testing the altered polypeptide for the desired activity.

### Anti-32144 Antibodies

Further disclosed is an anti-32144 antibody, or a fragment thereof (e.g., an antigen-binding fragment thereof). The term "antibody" as used herein refers to an immunoglobulin molecule or immunologically active portion thereof, i.e., an antigen-binding portion. As used herein, the term "antibody" refers to a protein comprising at least one, and preferably two, heavy (H) chain variable regions (abbreviated herein as VH), and at least one and preferably two light (L) chain variable regions (abbreviated herein as VL). The VH and VL regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat, E.A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917, which are incorporated herein by reference). Each VH and VL is composed of three CDR's and four FRs, arranged from amino-terminus to carboxy-terminus in the following order: FR1, CDR1, FR2, CDR2, FR3, CDR3, FR4.

The anti-32144 antibody can further include a heavy and light chain constant region, to thereby form a heavy and light immunoglobulin chain, respectively. In one embodiment, the antibody is a tetramer of two heavy immunoglobulin chains and two light immunoglobulin chains, wherein the heavy and light immunoglobulin chains are inter-connected by, e.g., disulfide bonds. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

As used herein, the term "immunoglobulin" refers to a protein consisting of one or more polypeptides substantially encoded by immunoglobulin genes. The recognized human immunoglobulin genes include the kappa, lambda, alpha (IgAl and IgA2), gamma (IgGl, IgG2, IgG3, IgG4), delta, epsilon and mu constant region genes, as well as the myriad immunoglobulin variable region genes. Full-length immunoglobulin "light chains" (about 25 KDa or 214 amino acids) are encoded by a variable region gene at the NH2-terminus (about 110 amino acids) and a kappa or lambda constant region gene at the COOH--terminus. Full-length immunoglobulin "heavy chains" (about 50 KDa or 446 amino acids), are similarly encoded by a variable region gene (about 116 amino acids) and one of the other aforementioned constant region genes, e.g., gamma (encoding about 330 amino acids).

The term "antigen-binding fragment" of an antibody (or simply "antibody portion," or "fragment"), as used herein, refers to one or more fragments of a full-length antibody that retain the ability to specifically bind to the antigen, e.g., 32144 polypeptide or fragment thereof. Examples of antigen-binding fragments of the anti-32144 antibody include, but are not limited to: (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CHl domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad Sci. USA 85:5879-5883). Such single chain antibodies are also encompassed within the term "antigen-binding fragment" of an antibody. These antibody fragments are obtained using conventional techniques known to those with skill in the art, and the fragments are screened for utility in the same manner as are intact antibodies.

The anti-32144 antibody can be a polyclonal or a monoclonal antibody. In other embodiments, the antibody can be recombinantly produced, e.g., produced by phage display or by combinatorial methods.

Phage display and combinatorial methods for generating anti-32144 antibodies are known in the art (as described in, e.g., Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 2:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 2:1373-1377; Hoogenboom et al. (1991) Nuc Acid Res 19:4133-4137; and Barbas et al. (1991) PNAS 88:7978-7982.

In one aspect, the anti-32144 antibody is a fully human antibody (e.g., an antibody made in a mouse which has been genetically engineered to produce an antibody from a human immunoglobulin sequence), or a non-human antibody, e.g., a rodent (mouse or rat), goat, primate (e.g., monkey), camel antibody. Preferably, the non-human antibody is a rodent (mouse or rat antibody). Method of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, e.g., Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:13 23 -13 26).

An anti-32144 antibody can be one in which the variable region, or a portion thereof, e.g., the CDR's, are generated in a non-human organism, e.g., a rat or mouse. Chimeric, CDR-grafted, and humanized antibodies are within the invention. Antibodies generated in a non-human organism, e.g., a rat or mouse, and then modified, e.g., in the variable framework or constant region, to decrease antigenicity in a human are within the invention.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura-et al., 1987, Canc. Res. 47:999-1005; Wood et al. (1985) Nature 314:446-449; and Shaw et al., 1988, J. Natl Cancer Inst. 80:1553-1559).

A humanized or CDR-grafted antibody will have at least one or two but generally all three recipient CDR's (of heavy and or light immuoglobulin chains) replaced with a donor CDR. The antibody may be replaced with at least a portion of a non-human CDR or only some of the CDR's may be replaced with non-human CDR's. It is only necessary to replace the number of CDR's required for binding of the humanized antibody to a 32144 or a fragment thereof Preferably, the donor will be a rodent antibody, e.g., a rat or mouse antibody, and the recipient will be a human framework or a human consensus framework. Typically, the immunoglobulin providing the CDR's is called the "donor" and the immunoglobulin providing the framework is called the "acceptor." In one aspect, the donor immunoglobulin is a non-human (e.g., rodent). The acceptor framework is a naturally-occurring (e.g., a human) framework or a consensus framework, or a sequence about 85% or higher, preferably 90%, 95%, 99% or higher identical thereto. As used herein, the term "consensus sequence" refers to the sequence formed from the most frequently occurring amino acids (or nucleotides) in a family of related sequences (See e.g., Winnaker, From Genes to Clones (Verlagsgesellschaft, Weinheim, Germany 1987). In a family of proteins, each position in the consensus sequence is occupied by the amino acid occurring most frequently at that position in the family. If two amino acids occur equally frequently, either can be included in the consensus sequence. A "consensus framework" refers to the framework region in the consensus immunoglobulin sequence.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. US 5,585,089, US 5,693,761 and US 5,693,762. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a 32144 polypeptide or fragment thereof The recombinant DNA encoding the humanized antibody, or fragment thereof, can then be cloned into an appropriate expression vector.

Humanized or CDR-grafted antibodies can be produced by CDR-grafting or CDR substitution, wherein one, two, or all CDR's of an immunoglobulin chain can be replaced. See e.g., U.S. Patent 5,225,539; Jones et al. 1986 Nature 321:552-525; Verhoeyan et al. 1988 Science 239:1534; Beidler et al. 1988 J. lmmunol. 141:4053-4060; Winter US 5,225,539. Winter describes a CDR-grafting method which may be used to prepare the humanized antibodies of the present invention (UK Patent Application GB 2188638A, filed on March 26, 1987; Winter US 5,225,539).

Also disclosed are humanized antibodies in which specific amino acids have been substituted, deleted or added. Preferred humanized antibodies have amino acid substitutions in the framework region, such as to improve binding to the antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR (see e.g., US 5,585,089). Criteria for selecting amino acids from the donor are described in US 5,585,089, e.g., columns 12-16 of US 5,585,089, the e.g., columns 12-16 of US 5,585,089. Other techniques for humanizing antibodies are described in Padlan et al. EP 519596 A1, published on December 23, 1992.

In preferred embodiments an antibody can be made by immunizing with purified 32144 antigen, or a fragment thereof, e.g., a fragment described herein, membrane associated antigen, tissue, e.g., crude tissue preparations, whole cells, preferably living cells, lysed cells, or cell fractions, e.g., membrane fractions.

A full-length 32144 protein or, antigenic peptide fragment of 32144 can be used as an immunogen or can be used to identify anti-32144 antibodies made with other immunogens, e.g., cells, membrane preparations, and the like. The antigenic peptide of 32144 should include at least-8-amino acid residues of the amino-acid sequence shown in SEQ ID NO:2 and encompasses an epitope of 32144. Preferably, the antigenic peptide includes at least 10 amino acid residues, more preferably at least 15 amino acid residues, even more preferably at least 20 amino acid residues, and most preferably at least 30 amino acid residues.

Fragments of 32144 can be used as immunogens or used to characterize the specificity of an antibody raised against a 32144 protein. Fragments of 32144 which include residues about 104 to 120, about 183 to 201, or about 415 to 438 can be used to make, e.g., used as immunogens or used to characterize the specificity of an antibody, antibodies against hydrophilic regions of the 32144 protein. Similarly, fragments of 32144 which include residues about 157 to 182, about 388 to 414, or about 471 to 489 can be used to make an antibody against a hydrophobic region of the 32144 protein; and fragments of 32144 which include residues about 69 to 289, about 204 to 235, or about 419 to 513 can be used to make an antibody against the fatty acid amide hydrolase region of the 32144 protein.

Antibodies reactive with, or specific for, any of these regions, or other regions or domains described herein are provided.

Antibodies can bind only native 32144 protein, only denatured or otherwise non-native 32144 protein, or both. Antibodies can bind linear or conformational epitopes. Conformational epitopes can sometimes be identified by identifying antibodies which bind to native but not denatured 32144 protein.

Preferred epitopes encompassed by the antigenic peptide are regions of 32144 are located on the surface of the protein, e.g., hydrophilic regions, as well as regions with high antigenicity. For example, an Emini surface probability analysis of the human 32144 protein sequence can be used to indicate the regions that have a particularly high probability of being localized to the surface of the 32144 protein and are thus likely to constitute surface residues useful for targeting antibody production.

Preferably the antibody can bind to the extracellular portion of the 32144 protein, e.g., it can bind to a whole cell which expresses the 32144 protein. Alterntively, the antibody binds an intracellular portion of the 32144 proteins. Preferably antibodies can bind one or more of purified antigen, membrane associated antigen, tissue, e.g., tissue sections, whole cells, preferably living cells, lysed cells, cell fractions, e.g., membrane fractions.

The anti-32144 antibody can be a single chain antibody. A single-chain antibody (scFV) may be engineered (see, for example, Colcher, D. et al. (1999) Ann N Y Acad Sci 880:263-80; and Reiter, Y. (1996) Clin Cancer Res 2:245-52). The single chain antibody can be dimerized or multimerized to generate multivalent antibodies having specificities for different epitopes of the same target 32144 protein.

Preferably the antibody has effector function and/or can fix complement. Alternatively the antibody does not recruit effector cells; or fix complement.

Preferably, the antibody has reduced or no ability to bind an Fc receptor. For example, it is a isotype or subtype, fragment or other mutant, which does not support binding to an Fc receptor, e.g., it has a mutagenized or deleted Fc receptor binding region.

Preferably, an anti-32144 antibody alters (e.g., increases or decreases) the fatty acid amide hydrolase activity of a 32144 polypeptide. For example, the antibody can bind at or in proximity to the active site, e.g., to an epitope that includes a residue located from about 204 to 235 of SEQ ID NO:2.

The antibody can be coupled to a toxin, e.g., a polypeptide toxin, e,g, ricin or diphtheria toxin or active fragment hereof, or a radioactive nucleus, or imaging agent, e.g. a radioactive, enzymatic, or other, e.g., imaging agent, e.g., a NMR contrast agent. Labels which produce detectable radioactive emissions or fluorescence are preferred.

An anti-32144 antibody (e.g., monoclonal antibody) can be used to isolate 32144 by standard techniques, such as affinity chromatography or immunoprecipitation. Moreover, an anti-32144 antibody can be used to detect 32144 protein (e.g., in a cellular lysate or cell supernatant) in order to evaluate the abundance and pattern of expression of the protein. Anti-32144 antibodies can be used diagnostically to monitor protein levels in tissue as part of a clinical testing procedure, e.g., to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include-umbelliferone,-fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Further disclosed is a nucleic acid which encodes an anti-32144 antibody, e.g., an anti-32144 antibody described herein. Also disclosed are vectors which include the nucleic acid and cells transformed with the nucleic acid, particularly cells which are useful for producing an antibody, e.g., mammalian cells, e.g. CHO or lymphatic cells.

Also disclosed are cell lines, e.g., hybridomas, which make an anti-32144 antibody, e.g., and antibody described herein, and method of using said cells to make a 32144 antibody.

### Recombinant Expression Vectors. Host Cells and Genetically Engineered Cells

Further disclosed are vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 32144 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 32144 proteins, mutant forms of 32144 proteins, fusion proteins, and the like).

The recombinant expression vectors can be designed for expression of 32144 proteins in prokaryotic or eukaryotic cells. For example, polypeptides of the invention can be expressed in *E*. *coli,* insect cells (e.g., using baculovirus expression vectors), yeast cells or mammalian cells. Suitable host cells are discussed further in Goeddel, (1990) Gene Expression Technology: Methods in Enzymology 185, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in *E*. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 32144 activity assays, (e.g., direct assays or competitive assays described in detail below), or to generate antibodies specific for 32144 proteins. Preferably, a fusion protein expressed in a retroviral expression vector can be used to infect bone marrow cells which are subsequently transplanted into irradiated recipients. The pathology of the subject recipient is then examined after sufficient time has passed (e.g., six weeks).

To maximize recombinant-protein expression in *E. coli* is-to express the protein in a host bacteria with an impaired capacity to proteolytically cleave the recombinant protein (Gottesman, S., (1990) Gene Expression Technology: Methods in Enzymology 185, Academic Press, San Diego, California 119-128). Another strategy is to alter the nucleic acid sequence of the nucleic acid to be inserted into an expression vector so that the individual codons for each amino acid are those preferentially utilized in *E*. *coli* (Wada et al., (1992) Nucleic Acids Res. 20:2111-2118). Such alteration of nucleic acid sequences of the invention can be carried out by standard DNA synthesis techniques.

The 32144 expression vector can be a yeast expression vector, a vector for expression in insect cells, e.g., a baculovirus expression vector or a vector suitable for expression in mammalian cells.

When used in mammalian cells, the expression vector's control functions can be provided by viral regulatory elements. For example, commonly used promoters are derived from polyoma, Adenovirus 2, cytomegalovirus and Simian Virus 40.

In another aspect, the promoter is an inducible promoter, e.g., a promoter regulated by a steroid hormone, by a polypeptide hormone (e.g., by means of a signal transduction pathway), or by a heterologous polypeptide (e.g., the tetracycline-inducible systems, "Tet-On" and "Tet-Off"; see, e.g., Clontech Inc., CA, Gossen and Bujard (1992) Proc. Natl. Acad Sci. USA 89:5547, and Paillard (1989) Human Gene Therapy 9:983)*.*

In another aspect, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto, and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) Science 249:374-379) and the α-fetoprotein promoter (Campes and Tilghman (1989) Genes Dev. 3:537-546).

Further disclosed is a recombinant expression vector comprising a 32144 DNA molecule cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus.

Further disclosed a host cell which includes a nucleic acid molecule described herein, e.g., a 32144 nucleic acid molecule within a recombinant expression vector or a 32144 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 32144 protein can be expressed in bacterial cells (such as *E*. *coli),* insect cells, yeast or mammalian cells (such as Chinese hamster ovary cells (CHO) or COS cells (African green monkey kidney cells CV-1 origin SV40 cells; Gluzman (1981) Celll23:175-182)) Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

A host cell can be used to produce (i.e., express) a 32144 protein. Accordingly, methods are disclosed for producing a 32144 protein using the host cells described herein. In one aspect, the method includes culturing the host cell of the invention (into which a recombinant expression vector encoding a 32144 protein has been introduced) in a suitable medium such that a 32144 protein is produced. In another aspect, the method further includes isolating a 32144 protein from the medium or the host cell.

Further disclosed is a cell or purified preparation of cells which include a 32144 transgene, or which otherwise misexpress 32144. The cell preparation can consist of human or non-human cells, e.g., rodent cells, e.g., mouse or rat cells, rabbit cells, or pig cells. In preferred embodiments, the cell or cells include a 32144 transgene, e.g., a heterologous form of a 32144, e.g., a gene derived from humans (in the case of a non-human cell). The 32144 transgene can be misexpressed, e.g., overexpressed or underexpressed. In other preferred embodiments, the cell or cells include a gene that mis-expresses an endogenous 32144, e.g., a gene the expression of which is disrupted, e.g., a knockout. Such cells can serve as a model for studying disorders that are related to mutated or mis-expressed 32144 alleles or for use in drug screening.

Further disclosed is a human cell, e.g., a hepatic or hematopoietic stem cell, transformed with nucleic acid which encodes a subject 32144 polypeptide.

Also disclosed are cells, preferably human cells, e.g., hematopoietic or fibroblast cells, in which an endogenous 32144 is under the control of a regulatory sequence that does not normally control the expression of the endogenous 32144 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 32144 gene. For example, an endogenous 32144 gene which is "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in e.g., Chappel, US 5,272,071; WO 91/06667, published in May 16, 1991.

Preferably, recombinant cells described herein can be used for replacement therapy in a subject. For example, a nucleic acid encoding a 32144 polypeptide operably linked to an inducible promoter (e.g., a steroid hormone receptor-regulated promoter) is introduced into a human or nonhuman, e.g., mammalian, e.g., porcine recombinant cell. The cell is cultivated and encapsulated in a biocompatible material, such as poly-lysine alginate, and subsequently implanted into the subject. See, e.g., Lanza (1996) Biotechnol. 14:1107; Joki et al. (2001) Nat. Biotechnol. 19:35; and U.S. Patent No. 5,876,742. Production of 32144 polypeptide can be regulated in the subject by administering an agent (e.g., a steroid hormone) to the subject. Alternatively, the implanted recombinant cells express and secrete an antibody specific for a 32144 polypeptide. The antibody can be any antibody or any antibody derivative described herein.

### Uses

The nucleic acid molecules, proteins, protein homologues, and antibodies described herein can be used in one or more of the following methods: a) screening assays; and b) predictive medicine (e.g., diagnostic assays, prognostic assays, monitoring clinical trials, and pharmacogenetics).

The isolated 32144 nucleic acid molecules can be used, for example, to express a 32144 protein (e.g., via a recombinant expression vector in a host cell in gene therapy applications), to detect a 32144 mRNA (e.g., in a biological sample) or a genetic alteration in a 32144 gene, and to modulate 32144 activity, as described further below. In addition, the 32144 proteins can be used to screen for naturally occurring 32144 substrates, to screen for drugs or compounds which modulate 32144 activity, as well as to treat disorders characterized by insufficient or excessive production of 32144 protein or production of 32144 protein forms which have decreased, aberrant or unwanted activity compared to 32144 wild type protein (e.g., cellular proliferation and/or differentiation disorders, neural disorders, metabolic or pain disorders, or sleep disorders). Moreover, the anti-32144 antibodies of the invention can be used to detect and isolate 32144 proteins, regulate the bioavailability of 32144 proteins, and modulate 32144 activity.

A method of evaluating a compound for the ability to interact with, e.g., bind, a subject 32144 polypeptide is provided. The method includes: contacting the compound with the subject 32144 polypeptide; and evaluating ability of the compound to interact with, e.g., to bind or form a complex with the subject 32144 polypeptide. This method can be performed in vitro, e.g., in a cell free system, or in vivo, e.g., in a two-hybrid interaction trap assay. This method can be used to identify naturally occurring molecules that interact with subject 32144 polypeptide. It can also be used to find natural or synthetic inhibitors of subject 32144 polypeptide. Screening methods are discussed in more detail below.

### Screening Assays

The invention provides methods (also referred to herein as "screening assays") for identifying modulators, i.e., candidate or test compounds or agents (e.g., proteins, peptides, peptidomimetics, peptoids, small molecules or other drugs) which bind to 32144 proteins, have a stimulatory or inhibitory effect on, for example, 32144 expression or 32144 activity, or have a stimulatory or inhibitory effect on, for example, the expression or activity of a 32144 substrate. Compounds thus identified can be used to modulate the activity of target gene products (e.g., 32144 genes) in a therapeutic protocol, to elaborate the biological function of the target gene product, or to identify compounds that disrupt normal target gene interactions.

In one embodiment, the invention provides assays for screening candidate or test compounds which are substrates of a 32144 protein or polypeptide or a biologically active portion thereof. In another embodiment, the invention provides assays for screening candidate or test compounds that bind to or modulate an activity of a 32144 protein or polypeptide or a biologically active portion thereof.

In one embodiment, an activity of a 32144 protein can be assayed by incubating a ¹⁴C-labeled fatty acid amide substrate with membrane extracts from cells that express a 32144 protein. Following incubation, the reaction products can be separated by thin layer chromatography, eluted from the silica with scintillation fluid, and counted in a scintillation counter. Assays such as this have been described, for example, in Cravatt et al. (1996), supra, and Giang and Cravatt (1997), supra.

The test compounds can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) J. Med Chem. 37:2678-85); spatially addressable parallel solid phase or solution phase libraries; synthetic library methods requiring deconvolution; the'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al. (1993) Proc. Natl. Acad Sci. U.S.A. 90:6909; Erb et al. (1994) Proc. Natl. Acad Sci. USA 91:11422; Zuckermann et al. (1994). J. Med Chem. 37:2678; Cho et al. (1993) Science 261:1303; Carrell et al. (1994) Angew. Chem. Int. Ed. Engl. 33:2059; Carell et al. (1994) Angew. Chem. Int. Ed Engl. 33:2061; and Gallop et al. (1994) J Med Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner, U.S. Patent No. 5,223,409), spores (Ladner U.S. Patent No. 5,223,409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra.).* '

In one embodiment, an assay is a cell-based assay in which a cell which expresses a 32144 protein or biologically active portion thereof is contacted with a test compound, and the ability of the test compound to modulate 32144 activity is determined. Determining the ability of the test compound to modulate 32144 activity can be accomplished by monitoring, for example, fatty acid amide hydrolase activity. The cell, for example, can be of mammalian origin, e.g., human.

The ability of the test compound to modulate 32144 binding to a compound, e.g., a 32144 substrate, or to bind to 32144 can also be evaluated. This can be accomplished, for example, by coupling the compound, e.g., the substrate, with a radioisotope or enzymatic label such that binding of the compound, e.g., the substrate, to 32144 can be determined by detecting the labeled compound, e.g., substrate, in a complex. Alternatively, 32144 could be coupled with a radioisotope or enzymatic label to monitor the ability of a test compound to modulate 32144 binding to a 32144 substrate in a complex. For example, compounds (e.g., 32144 substrates) can be labeled with ¹²⁵I_{,} ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 32144 substrate) to interact with 32144 with or without the labeling of any of the interactants can be evaluated. For example, a microphysiometer can be used to detect the interaction of a compound with 32144 without the labeling of either the compound or the 32144. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 32144.

In yet another embodiment, a cell-free assay is provided in which a 32144 protein or biologically active portion thereof is contacted with a test compound and the ability of the test compound to bind to the 32144 protein or biologically active portion thereof is evaluated. Preferred biologically active portions of the 32144 proteins to be used in assays of the present invention include fragments which participate in interactions with non-32144 molecules, e.g., fragments with high surface probability scores.

Soluble and/or membrane-bound forms of isolated proteins (e.g., 32144 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 32144 protein to bind to a target molecule can be accomplished using real-time Biomolecular Interaction Analysis (BIA) (see, e.g., Sjolander, S. and Urbaniczky, C. (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 32144, an anti-32144 antibody or its target molecule to facilitate separation of complexed from uncomplexed forms of one or both of the proteins, as well as to accommodate automation of the assay. Binding of a test compound to a 32144 protein, or interaction of a 32144 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/32144 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 32144 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 32144 binding or activity determined using standard techniques.

Other techniques for immobilizing either a 32144 protein or a target molecule on matrices include using conjugation of biotin and streptavidin. Biotinylated 32144 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 32144 protein or target molecules but which do not interfere with binding of the 32144 protein to its target molecule. Such antibodies can be derivatized to the wells of the plate, and unbound target or 32144 protein trapped in the wells by antibody conjugation. Methods for detecting such complexes, in addition to those described above for the GST-immobilized complexes, include immunodetection of complexes using antibodies reactive with the 32144 protein or target molecule, as well as enzyme-linked assays which rely on detecting an enzymatic activity associated with the 32144 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) Trends Biochem Sci 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) Current Protocols in Molecular Biology, J. Wiley: New York). Such resins and chromatographic techniques are known to one skilled in the art (see, e.g., Heegaard, N.H., (1998) J Mol Recognit 11:141-8; Hage, D.S., and Tweed, S.A. (1997) J Chromatogr B Biomed Sci Appl. 699:499-525). Further, fluorescence energy transfer may also be conveniently utilized, as described herein, to detect binding without further purification of the complex from solution.

In a preferred embodiment, the assay includes contacting the 32144 protein or biologically active portion thereof with a known compound which binds 32144 to form an assay mixture, contacting the assay mixture with a test compound, and determining the ability of the test compound to interact with a 32144 protein, wherein determining the ability of the test compound to interact with a 32144 protein includes determining the ability of the test compound to preferentially bind to 32144 or biologically active portion thereof, or to modulate the activity of a target molecule, as compared to the known compound.

The target gene products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 32144 genes herein identified. In an alternative embodiment, the invention provides methods for determining the ability of the test compound to modulate the activity of a 32144 protein through modulation of the activity of a downstream effector of a 32144 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 32144 proteins can be used as "bait proteins" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with 32144 ("32144-binding proteins" or "32144-bp") and are involved in 32144 activity. Such 32144-bps can be activators or inhibitors of signals by the 32144 proteins or 32144 targets as, for example, downstream elements of a 32144-mediated signaling pathway.

The two-hybrid system is based on the modular nature of most transcription factors, which consist of separable DNA-binding and activation domains. Briefly, the assay utilizes two different DNA constructs. In one construct, the gene that codes for a 32144 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 32144 protein can be the fused to the activator domain.) If the "bait" and the "prey" proteins are able to interact, *in vivo,* forming a 32144-dependent complex, the DNA-binding and activation domains of the transcription factor are brought into close proximity. This proximity allows transcription of a reporter gene (e.g., lacZ)-which-is-operably linked to a transcriptional regulatory site responsive to the transcription factor. Expression of the reporter gene can be detected and cell colonies containing the functional transcription factor can be isolated and used to obtain the cloned gene which encodes the protein which interacts with the 32144 protein.

In another embodiment, modulators of 32144 expression are identified. For example, a cell or cell free mixture is contacted with a candidate compound and the expression of 32144 mRNA or protein evaluated relative to the level of expression of 32144 mRNA or protein in the absence of the candidate compound. When expression of 32144 mRNA or protein is greater in the presence of the candidate compound than in its absence, the candidate compound is identified as a stimulator of 32144 mRNA or protein expression. Alternatively, when expression of 32144 mRNA or protein is less (statistically significantly less) in the presence of the candidate compound than in its absence, the candidate compound is identified as an inhibitor of 32144 mRNA or protein expression. The level of 32144 mRNA or protein expression can be determined by methods described herein for detecting 32144 mRNA or protein.

In another aspect, the invention pertains to a combination of two or more of the assays described herein. For example, a modulating agent can be identified using a cell-based or a cell free assay, and the ability of the agent to modulate the activity of a 32144 protein can be confirmed *in vivo,* e.g., in an animal such as an animal model for a cellular proliferation and/or differentiation disorder, a neural disorder, a metabolic or pain disorder, or a sleep disorder.

An agent identified as described herein (e.g., a 32144 modulating agent, an antisense 32144 nucleic acid molecule, a 32144-specific antibody, or a 32144-binding partner) can be used in an appropriate animal model to determine the efficacy, toxicity, side effects, or mechanism of action, of treatment with such an agent. Furthermore, agents identified by the above-described screening assays can be used for treatments as described herein.

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 32144..

Such disorders include, e.g., a disorder associated with the misexpression of 32144 gene, such as a cellular proliferation and/or differentiation disorder, e.g., lung, colon, or ovarian cancer.

The method includes one or more of the following:
detecting, in a tissue of the subject, the presence or absence of a mutation which affects the expression of the 32144 gene, or detecting the presence or absence of a mutation in a region which controls the expression of the gene, e.g., a mutation in the 5' control region;
detecting, in a tissue of the subject, the presence or absence of a mutation which alters the structure of the 32144 gene;
detecting, in a tissue of the subject, the misexpression of the 32144 gene, at the mRNA level, e.g., detecting a non-wild type level of a mRNA ;
detecting, in a tissue of the subject, the misexpression of the gene, at the protein level, e.g., detecting a non-wild type level of a 32144 polypeptide.

In preferred embodiments the method includes: ascertaining the existence of at least one of a deletion of one or more nucleotides from the 32144 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, or naturally occurring mutants thereof or 5' or 3' flanking sequences naturally associated with the 32144 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of an alteration in the level of a messenger RNA transcript of the 32144 gene; the presence of a non-wild type splicing pattern of a messenger RNA transcript of the gene; or a non-wild type level of 32144.

Methods of the invention can be used prenatally or to determine if a subject's offspring will be at risk for a disorder.

In preferred embodiments the method includes determining the structure of a 32144 gene, an abnormal structure being indicative of risk for the disorder.

In preferred embodiments the method includes contacting a sample from the subject with an antibody to the 32144 protein or a nucleic acid, which hybridizes specifically with the gene. These and other embodiments are discussed below.

### Diagnostic and Prognostic Assays

Diagnostic and prognostic assays of the invention include method for assessing the expression level of 32144 molecules and for identifying variations and mutations in the sequence of 32144 molecules.

**Expression Monitoring and Profiling.** The presence, level, or absence of 32144 protein or nucleic acid in a biological sample can be evaluated by obtaining a biological sample from a test subject and contacting the biological sample with a compound or an agent capable of detecting 32144 protein or nucleic acid (e.g., mRNA, genomic DNA) that encodes 32144 protein such that the presence of 32144 protein or nucleic acid is detected in the biological sample. The term "biological sample" includes tissues, cells and biological fluids isolated from a subject, as well as tissues, cells and fluids present within a subject. A preferred biological sample is serum. The level of expression of the 32144 gene can be measured in a number of ways, including, but not limited to: measuring the mRNA encoded by the 32144 genes; measuring the amount of protein encoded by the 32144 genes; or measuring the activity of the protein encoded by the 32144 genes.

The level of mRNA corresponding to the 32144 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 32144 nucleic acid, such as the nucleic acid of SEQ ID NO:1, or a portion thereof, such as an oligonucleotide of at least 7, 15, 30, 50, 100, 250 or 500 nucleotides in length and sufficient to specifically hybridize under stringent conditions to 32144 mRNA or genomic DNA. The probe can be disposed on an address of an array, e.g., an array described below. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array described below. A skilled artisan can adapt known mRNA detection methods for use in detecting the level of mRNA encoded by the 32144 genes.

The level of mRNA in a sample that is encoded by one of 32144 can be evaluated with nucleic acid amplification, e.g., by rtPCR (Mullis (1987) U.S. Patent No. 4,683,202), ligase chain reaction (Barany (1991) Proc. Natl. Acad. Sci. USA 88:189-193), self sustained sequence replication (Guatelli et al., (1990) Proc. Natl. Acad Sci. USA 87:1874-1878), transcriptional amplification system (Kwoh et al., (1989), Proc. Natl. Acad. Sci. USA 86:1173-1177), Q-Beta Replicase (Lizardi et al., (1988) Bio/Technology 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 32144 gene being analyzed.

In another embodiment, the methods further contacting a control sample with a compound or agent capable of detecting 32144 mRNA, or genomic DNA, and comparing the presence of 32144 mRNA or genomic DNA in the control sample with the presence of 32144 mRNA or genomic DNA in the test sample. In still another embodiment, serial analysis of gene expression, as described in U.S. Patent No. 5,695,937, is used to detect 32144 transcript levels.

A variety of methods can be used to determine the level of protein encoded by 32144. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 32144 protein in a biological sample *in vitro* as well as *in vivo. In vitro* techniques for detection of 32144 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 32144 protein include introducing into a subject a labeled anti-32144 antibody. For example, the antibody can be labeled with a radioactive marker whose presence and location in a subject can be detected by standard imaging techniques. In another embodiment, the sample is labeled, e.g., biotinylated and then contacted to the antibody, e.g., an anti-32144 antibody positioned on an antibody array (as described below). The sample can be detected, e.g., with avidin coupled to a fluorescent label.

In another embodiment, the methods further include contacting the control sample with a compound or agent capable of detecting 32144 protein, and comparing the presence of 32144 protein in the control sample with the presence of 32144 protein in the test sample.

Also disclosed are kits for detecting the presence of 32144 in a biological sample. For example, the kit can include a compound or agent capable of detecting 32144 protein or mRNA in a biological sample; and a standard. The compound or agent can be packaged in a suitable container. The kit can further comprise instructions for using the kit to detect 32144 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 32144 expression or activity, e.g., lung, colon, breast, or ovarian cancer. As used herein, the term "unwanted" includes an unwanted phenomenon involved in a biological response such as a neural disorder or deregulated cell proliferation.

In one embodiment, a disease or disorder associated with aberrant or unwanted 32144 expression or activity is identified. A test sample is obtained from a subject and 32144 protein or nucleic acid (e.g., mRNA or genomic DNA) is evaluated, wherein the level, e.g., the presence or absence, of 32144 protein or nucleic acid is diagnostic for a subject having or at risk of developing a disease or disorder associated with aberrant or unwanted 32144 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 32144 expression or activity. For example, such methods can be used to determine whether a subject can be effectively treated with an agent for a neuronal disorder, a sleep disorder, a metabolic or pain disorder, or a cellular proliferation and/or differentiation disorder.

Also disclosed is a computer medium having a plurality of digitally encoded data records. Each data record includes a value representing the level of expression of 32144 in a sample, and a descriptor of the sample. The descriptor of the sample can be an identifier of the sample, a subject from which the sample was derived (e.g., a patient), a diagnosis, or a treatment (e.g., a preferred treatment). In a preferred embodiment, the data record further includes values representing the level of expression of genes other than 32144 (e.g., other genes associated with a 32144-disorder, or other genes on an array). The data record can be structured as a table, e.g., a table that is part of a database such as a relational database (e.g., a SQL database of the Oracle or Sybase database environments).

Also featured is a method of evaluating a sample. The method includes providing a sample, e.g., from the subject, and determining a gene expression profile of the sample, wherein the profile includes a value representing the level of 32144 expression. The method can further include comparing the value or the profile (i.e., multiple values) to a reference value or reference profile. The gene expression profile of the sample can be obtained by any of the methods described herein (e.g., by providing a nucleic acid from the sample and contacting the nucleic acid to an array). The method can be used to diagnose a cellular proliferation and/or differentiation disorder in a subject wherein an increase in 32144 expression is an indication that the subject has or is disposed to having a cellular proliferation and/or differentiation disorder. The method can be used to monitor a treatment for cellular proliferation and/or differentiation disorder in a subject. For example, the gene expression profile can be determined for a sample from a subject undergoing treatment. The profile can be compared to a reference profile or to a profile obtained from the subject prior to treatment or prior to onset of the disorder (see, e.g., Golub et al. (1999) Science 286:531).

In yet another aspect, the invention features a method of evaluating a test compound (see also, "Screening Assays", above). The method includes providing a cell and a test compound; contacting the test compound to the cell; obtaining a subject expression profile for the contacted cell; and comparing the subject expression profile to one or more reference profiles. The profiles include a value representing the level of 32144 expression. In a preferred embodiment, the subject expression profile is compared to a target profile, e.g., a profile for a normal cell or for desired condition of a cell. The test compound is evaluated favorably if the subject expression profile is more similar to the target profile than an expression profile obtained from an uncontacted cell.

In another aspect, the invention features, a method of evaluating a subject. The method includes: a) obtaining a sample from a subject, e.g., from a caregiver, e.g., a caregiver who obtains the sample from the subject; b) determining a subject expression profile for the sample. Optionally, the method further includes either or both of steps: c) comparing the subject expression profile to one or more reference expression profiles; and d) selecting the reference profile most similar to the subject reference profile. The subject expression profile and the reference profiles include a value representing the level of 32144 expression. A variety of routine statistical measures can be used to compare two reference profiles. One possible metric is the length of the distance vector that is the difference between the two profiles. Each of the subject and reference profile is represented as a multi-dimensional vector, wherein each dimension is a value in the profile.

The method can further include transmitting a result to a caregiver. The result can be the subject expression profile, a result of a comparison of the subject expression profile with another profile, a most similar reference profile, or a descriptor of any of the aforementioned. The result can be transmitted across a computer network, e.g., the result can be in the form of a computer transmission e.g., a computer data signal embedded in a carrier wave.

Also disclosed is a computer medium having executable code for effecting the following steps: receive a subject expression profile; access a database of reference expression profiles; and either i) select a matching reference profile most similar to the subject expression profile or ii) determine at least one comparison score for the similarity of the subject expression profile to at least one reference profile. The subject expression profile, and the reference expression profiles each include a value representing the level of 32144 expression.

This invention is further illustrated by the following examples that should not be construed as limiting.

### EXAMPLES

### Example 1: Identification and Characterization of Human 32144 cDNA

The human 32144 nucleic acid sequence is recited as follows:

GCACTGGACTTGTAAACGAAAAGCTTCATAAGTCCCTCTTTGCTTAGTACTT
TTCTCGTCCTTTCCCCAGGGTGCACGTAACCCTCAAGCACTAGGACCGTGCGGAAT
CCAGGCTGCGATGGCACCTTCATTTACCGCCCGCATTCAGTTGTTCCTCTTGCGGGC
GCTAGGCTTTCTCATAGGCTTAGTAGGCCGAGCAGCTTTAGTCTTAGGGGGTCCAA
AGTTTGCCTCAAAGACCCCTCGGCCGGTGACTGAACCATTGCTTCTGCTTTCGGGGA
TGCAGCTGGCCAAGCTGATCCGACAGAGAAAGGTGAAATGTATAGATGTTGTTCAG
GCTTATATCAACAGAATCAAGGACGTGAACCCAATGATCAATGGAATTGTCAAGTA
CAGGTTTGAGGAAGCGATGAAGGAGGCTCATGCTGTAGATCAAAAGCTTGCAGAG
AAGCAGGAAGATGAAGCCACCCTGGAAAATAAATGGCCCTTCCTTGGGGTTCCTTT
GACAGTCAAGGAAGCTTTCCAGCTACAAGGAATGCCCAATTCTTCTGGACTCATGA
ACCGTCGTGATGCCATTGCCAAAACAGATGCCACTGTGGTGGCATTACTGAAGGGA
GCTGGTGCCATTCCTCTTGGCATAACCAACTGTAGTGAGTTGTGTATGTGGTATGAA
TCCAGTAACAAGATCTATGGCCGATCAAACAACCCATATGATTTACAGCATATTGT
AGGTGGAAGTTCTGGTGGTGAGGGCTGCACACTGGCAGCTGCCTGCTCAGTTATTG
GTGTGGGCTCTGATATTGGTGGTAGCATTCGAATGCCTGCTTTCTTCAATGGTATAT
TTGGACACAAGCCTTCTCCAGGTGTGGTTCCCAACAAAGGTCAGTTTCCCTTGGCTG
TGGGAGCCCAGGAGTTGTTTCTGTGCACTGGTCCTATGTGCCGCTATGCTGAAGAC
CTGGCCCCCATGTTGAAGGTCATGGCAGGACCTGGGATCAAAAGGTTAAAACTAGA
CACAAAGGTACATTTAAAAGACTTAAAATTTTACTGGATGGAACATGATGGAGGCT

CATTTTTAATGTCCAAAGTGGACCAAGATCTCATTATGACTCAGAAAAAGGTTGTG
GTTCACCTTGAAACTATTCTAGGAGCCTCAGTTCAACATGTTAAACTGAAGAAAAT
GAAGTACTCTTTTCAGTTGTGGATCGCAATGATGTCAGCAAAGGACATGATGGGA
AGGAACCTGTGAAATTTGTAGATTTGCTTGGTGACCATGGGAAACATGTCAGTCCT
CTGTGGGAGTTGATCAAATGGTGCCTGGGTCTGTCAGTGTACACCATCCCTTCCATT
GGACTGGCTTTGTTGGAAGAAAAGCTCAGATATAGCAATGAGAAATACCAAAAGT
TTAAGGCAGTGGAAGAAAGCCTGCGTAAAGAGCTGGTGGATATGCTAGGTGATGA
TGGTGTGTTCTTATATCCCTCACATCCCACAGTGGCACCTAAGCATCATGTCCCTCT
AACACGGCCCTTCAACTTTGCTTACACAGGTGTCTTCAGTGCCCTGGGTTTGCCTGT
GACCCAATGCCCACTGGGACTGAATGCCAAAGGACTCCCTTTAGGCATCCAGGTTG
TGGCTGGACCCTTTAATGATCATCTGACCCTGGCTGTGGCCCAGTACTTGGAGAAA
ACTTTTGGGGGCTGGGTCTGTCCAGGAAAGTTTTAGGAGGACCTTCTGCAAGGTTA
ATGTGTGTGTGTGTTTGTGTTCGTGTGGTGGTGTTTCTATTAATTGGGTGAAACCAA
GCACCAGCAGACAAGCAGAGAAACAACTGGGGAATTTATTGACTCATTTAGTTATT
CTTTCTACTTTTATTTCCTTCTCTAACTGTTGGTCTTACTAAAATGGTAATATTTGCT
TCTTGCTTTTATGTTACTGGAAAATTAGGACATGTAAATGGATAAGTGCAATAAAG
TTTCCTAAATGCTGAAAAAAAAAAAAAAAAAAAGGCCGC (SEQ ID NO:1).

The human 32144 sequence (Fig. 1; SEQ ID NO:1), which is approximately 2007 nucleotides long. The nucleic acid sequence includes an initiation codon (ATG) and a termination codon (TAG) which are bolded and underscored above. The region between and inclusive of the initiation codon and the termination codon is a methionine-initiated coding sequence of about 1599 nucleotides, including the termination codon (nucleotides indicated as "coding" of SEQ ID NO:1; SEQ ID NO:3). The coding sequence encodes a 532 amino acid protein (SEQ ID NO:2), which is recited as follows:

MAPSFTARIQLFLLRALGFLIGLVGRAALVLGGPKFASKTPRPVTEPLLLLSGMQ
LAKLIRQRKVKCIDVVQAYINRIKDVNPMINGIVKYRFEEAMKEAHAVDQKLAEKQED
EATLENKWPFLGVPLTVKEAFQLQGMPNSSGLMNRRDAIAKTDATVVALLKGAGAIPL
GITNCSELCMWYESSNKIYGRSNNPYDLQHIVGGSSGGEGCTLAACSVIGVGSDIGGSI
RMPAFFNGIFGHKPSPGVVPNKGQFPLAVGAQELFLCTGPMCRYAEDLAPMLKVMAG
PGIKRLKLDTKVHLKDLKFYWMEHDGGSFLMSKVDQDLIMTQKKVVVHLETILGASV
QHVKLKKMKYSFQLWIAMMSAKGHDGKEPVKFVDLLGDHGKHVSPLWELIKWCLGL

SVYTIPSIGLALLEEKLRYSNEKYQKFKAVEESLRKELVDMLGDDGVFLYPSHPTVAPK
HHVPLTRPFNFAYTGVFSALGLPVTQCPLGLNAKGLPLGIQVVAGPFNDHLTLAVAQY
LEKTFGGWVCPGKF (SEQ ID NO:2).

### Example 2: Tissue Distribution of 32144 mRNA

Endogenous human 32144 gene expression was determined using the Perkin-Elmer/ABI 7700 Sequence Detection System which employs TaqMan technology. Briefly, TaqMan technology relies on standard RT-PCR with the addition of a third gene-specific oligonucleotide (referred to as a probe) which has a fluorescent dye coupled to its 5' end (typically 6-FAM) and a quenching dye at the 3' end (typically TAMRA). When the fluorescently tagged oligonucleotide is intact, the fluorescent signal from the 5' dye is quenched. As PCR proceeds, the 5' to 3' nucleotytic activity of Taq polymerase digests the labeled primer, producing a free nucleotide labeled with 6-FAM, which is now detected as a fluorescent signal. The PCR cycle where fluorescence is first released and detected is directly proportional to the starting amount of the gene of interest in the test sample, thus providing a quantitative measure of the initial template concentration. Samples can be internally controlled by the addition of a second set of primers/probe specific for a housekeeping gene such as GAPDH which has been labeled with a different fluorophore on the 5' end (typically VIC).

To determine the level of 32144 in various human tissues a primer/probe set was designed. Total RNA was prepared from a series of human tissues using an RNeasy kit from Qiagen. First strand cDNA was prepared from 1 µg total RNA using an oligo-dT primer and Superscript II reverse transcriptase (Gibco/BRL). cDNA obtained from approximately 50 ng total RNA was used per TaqMan reaction. Tissues tested include the human tissues and several cell lines shown in Tables 1-5. 32144 mRNA was detected in a number of tissues, including the kidney, pancreas, brain, and liver (Table 1). Importantly, 32144 expression was upregulated in most of the lung, colon, breast, and ovarian tumors tested (Tables 1-3). 32144 mRNA was also detected in several tumor cell lines, whether grown in vivo (Table 4) or in vitro (Table 5), and growth of breast tumor cell lines on agar correlated with increased expression of 32144 mRNA as compared to growth on plastic (Table 5).

The incidence of tumor-associated expression of 32144 mRNA in lung, ovary, breast, and colon tissues was further evaluated by in situ hybridization (Table 6). Notable tumor-associated expression of 32144 is seen in all of the different tumor types tested. This data, like the Taqman data, suggests a role for 32144 in tumor development. In addition, expression of 32144 mRNA in invasive indolent breast carcinomas vs. metastatic breast carcinomas was evaluated by hybridizing tumor cell RNA to microarray chips that were capable of detecting 32144 nucleic acids (Table 7). All of the tumors tested expressed 32144 mRNA, while 2/5 metastatic tumors and 0/3 invasive indolent tumors displayed an relative increase in 32144 expression. This data, along with the colon tumor in-situ hybridization data reveals a positive correlation between 32144 expression and tumor metastasis, at least for breast and colon tumors.

**Table 1**

| **Tissue Type** | **32144 Mean** | **β2 Mean** | **Relative Expression** |
|---|---|---|---|
| Artery normal | 34.72 | 22.38 | 0.19 |
| Aorta diseased | 33.33 | 23.00 | 0.78 |
| Vein normal | 35.26 | 20.66 | 0.00 |
| Coronary SMC | 35.06 | 21.18 | 0.00 |
| HUVEC | 31.24 | 21.50 | 1.17 |
| Hemangioma | 31.99 | 20.05 | 0.25 |
| Heart normal | 33.42 | 20.90 | 0.17 |
| HeartCHF | 31.20 | 20.26 | 0.51 |
| Kid ney | 27.57 | 20.53 | 7.60 |
| Skeletal Muscle | 40.00 | 28.39 | 0.00 |
| Adipose normal | 37.55 | 29.80 | 0.00 |
| Pancreas | 28.33 | 22.43 | 16.69 |
| primary osteoblasts | 35.35 | 21.07 | 0.00 |
| Osteoc lasts (diff) | 34.77 | 17.92 | 0.01 |
| Skin normal | 31.45 | 22.34 | 1.81 |
| Spinal cord normal | 32.28 | 21.36 | 0.52 |
| Bra in Cortex normal | 29.65 | 22.56 | 7.37 |
| Brain Hypothalamus normal | 30.93 | 22.57 | 3.02 |
| Ne rve | 34.01 | 22.24 | 0.29 |
| DRG (Dorsal Root Ganglion) | 33.84 | 22.35 | 0.35 |
| Breast normal | 30.32 | 21.55 | 2.28 |
| Breast tumor | 28.50 | 20.88 | 5.05 |
| Ovary normal | 29.64 | 20.07 | 1.32 |
| Ovary Tumor | 29.09 | 20.02 | 1.85 |
| Salivary glands | 28.74 | 19.91 | 2.19 |
| Colon normal | 28.41 | 18.40 | 0.97 |
| Colon Tumor | 28.68 | 21.98 | 9.62 |
| Lung normal | 28.62 | 18.32 | 0.79 |
| Lung tumor | 25.91 | 20.45 | 22.72 |
| Lung COPD | 28.17 | 18.70 | 1.41 |
| Colon IBD | 27.64 | 18.00 | 1.26 |
| Liver normal | 27.31 | 20.34 | 7.98 |
| Liver fibrosis | 27.41 | 20.89 | 10.82 |
| Spleen normal | 30.79 | 19.91 | 0.53 |
| Tonsil normal | 25.54 | 17.77 | 4.60 |
| Lymph node normal | 28.48 | 19.77 | 2.40 |
| Sma II intestine normal | 30.61 | 20.50 | 0.91 |
| Macrophages | 31.80 | 17.40 | 0.05 |
| Synovium | 31.52 | 19.78 | 0.29 |
| BM-MNC | 33.83 | 19.09 | 0.04 |
| Activated PBMC | 28.09 | 18.29 | 1.12 |
| Neutrophils | 36.31 | 19.26 | 0.00 |
| Megakaryocytes | 32.13 | 19.11 | 0.12 |
| Erythroid | 33.20 | 22.05 | 0.44 |
| positive control | 28.92 | 20.59 | 3.12 |

As shown in the "Relative Expression" column of Table 1, 32144 mRNA is expressed in the pancreas, kidney, liver, cerebral cortex, hypothalamus, tonsils, lymph nodes, breast, salivary gland, skin, and ovary. Weak expression is observed in the heart and blood vessels, dorsal root gaglia, colon, lung, spleen, small intestine, and blood cells. In addition, 32144 expression is highly upregulated in lung, colon, and breast tumors, and slightly upregulated in ovarian tumors. Abbreviations used in Table 1: SMC, smooth muscle cell; HUVEC, human umbilical vein endothelial cells; CHF, congestive heart failure; diff, differentiated; COPD, chronic obstructive pulmonary disease; IBD, inflammatory bowel disease; BM-MNC, bone marrow mononuclear cell; PBMC, pre-bone marrow cell.

**Table 2**

| **Tissue Type** | **32144.1 Mean** | **β2 Mean** | **Relative Expression** |
|---|---|---|---|
| PIT400 Breast N | 31.85 | 20.18 | 0.31 |
| PIT 372 Breast N | 32.09 | 20.92 | 0.43 |
| PIT 271 Breast N | 35.4 | 25.48 | 0.00 |
| MDA 106 Breast T | 31.93 | 21.11 | 0.55 |
| MDA 234 Breast T | 29.27 | 18.77 | 0.69 |
| NDR 57 Breast T | 30.98 | 19.75 | 0.41 |
| MDA 304 Breast T | 29.8 | 19.34 | 0.71 |
| NDR 58 Breast T | 26.43 | 17.88 | 2.66 |
| NDR 132 Breast T | 30.15 | 21.54 | 2.57 |
| NDR 07 Breast T | 30 | 19.65 | 0.76 |
| NDR 12 Breast T | 28.84 | 21.69 | 7.02 |
| PIT 208 Ovary N | 32.35 | 19.52 | 0.14 |
| CHT 620 Ovary N | 34.23 | 20.1 | 0.06 |
| CHT 619 Ovary N | 34.7 | 20.6 | 0.06 |
| CLN 03 Ovary T | 29.57 | 20.08 | 1.39 |
| CLN 17 Ovary T | 27.84 | 20.35 | 5.58 |
| CLN 07 Ovary T | 30.43 | 19.66 | 0.57 |
| CLN 08 Ovary T | 29.87 | 18.9 | 0.50 |
| MDA 216 Ovary T | 33.05 | 21.04 | 0.24 |
| CLN 012 Ovary T | 31.83 | 22.16 | 1.22 |
| MDA 25 Ovary T | 29.89 | 22.62 | 6.50 |
| MDA 183 Lung N | 30.52 | 18.43 | 0.23 |
| CLN 930 Lung N | 32.03 | 19.36 | 0.15 |
| MDA 185 Lung N | 34.11 | 19.88 | 0.05 |
| CHT 816 Lung N | 30.36 | 17.2 | 0.11 |
| MPI 215 Lung T--SmC | 31.25 | 18.91 | 0.19 |
| MDA 259 Lung T-PDNSCCL | 27.37 | 19.87 | 5.52 |
| CHT 832 Lung T-PDNSCCL | 28.59 | 19.36 | 1.68 |
| MDA 253 Lung T-PDNSCCL | 29.66 | 19 | 0.62 |
| CHT 911 Lung T-SCC | 28.73 | 19.3 | 1.45 |
| CHT 793 Lung T-ACA (?) | 29.3 | 19.2 | 0.91 |
| MDA 262 Lung T-SCC | 31.18 | 23.35 | 4.38 |
| CHT 211 Lung T-AC | 28.01 | 19.86 | 3.53 |
| NHBE | 30.45 | 21.66 | 2.27 |
| MDA 127 N Ovarian Epithelial Cells | 34.08 | 16.97 | 0.01 |
| MDA 224 N Ovarian Epithelial Cells | 36.37 | 16.62 | 0.00 |
| MDA 124 Ovarian Ascites | 28.13 | 15.52 | 0.16 |
| MDA 126 Ovarian Ascites | 26.59 | 17.4 | 1.71 |

As shown in the "Relative Expression" column of Table 2, 32144 mRNA expression is slightly upregulated in 4/8 of the breast tumor samples tested, as compared to normal breast tissue, and dramatically upregulated in 3/8 of the breast tumor samples. Likewise, 7/7 ovary tumor samples displayed an increase in 32144 expression relative to normal ovary tissue, while 2/7 contained dramatically upregulated levels of 32144 mRNA. Amongst lung tumor samples tested, 8/9 displayed an increase in 32144 expression relative to normal lung tissue, with 4/9 containing highly elevated levels of 32144 mRNA. Abbreviations used in Table 2: N, normal tissue; T, tumor; SmC, small cell carcinoma; PDNSCCL, poorly differentiated non-small cell carcinoma; SCC, squamous cell carcinoma; AC, adenocarcinoma; NHBE, lung cell line.

**Table 3**

| **Tissue Type** | **32144.1 Mean** | **β2 Mean** | **Relative Expression** |
|---|---|---|---|
| CHT 523 Colon N | 31.64 | 19 | 0.16 |
| NDR 104 Colon N | 28.39 | 19.09 | 1.59 |
| CHT 416 Colon N | 30.23 | 19.48 | 0.58 |
| CHT 452 Colon N | 32 | 18.7 | 0.10 |
| NDR 210 Colon T | 32.7 | 24.07 | 2.51 |
| CHT 398 Colon T | 27.32 | 19.91 | 5.84 |
| CHT 382 Colon T | 26.62 | 18.97 | 4.96 |
| CHT 944 Colon T | 28.61 | 18.84 | 1.15 |
| CHT 528 Colon T | 26.5 | 19.07 | 5.78 |
| CHT 1365 Colon T | 27.56 | 19.2 | 3.05 |
| CHT 372 Colon T | 30.31 | 20.27 | 0.95 |
| CLN 609 Colon T | 28.55 | 20.02 | 2.70 |
| CHT 01 Liver Met | 28.29 | 18.11 | 0.87 |
| NDR 100 Liver Met | 26.37 | 18.59 | 4.53 |
| CHT 340 Liver Met | 30.07 | 20.79 | 1.60 |
| NDR 217 Liver Met | 29.04 | 19.2 | 1.10 |
| PIT 260 Liver N | 27.5 | 17.77 | 1.17 |
| CHT 320 Liver N | 30.67 | 23.56 | 7.21 |
| C48 HMVEC-Prol | 36.87 | 20.74 | 0.00 |
| ONC 102 Hemangioma | 33.59 | 20.15 | 0.09 |

As shown in the "Relative Expression" column of Table 3, 6/8 of the tested colon tumors had an elevated level of 32144 expression as compared to normal colon tissue, with 3/8 displaying a dramatic increase in 32144 mRNA expression. All liver metastases tested expressed 32144 mRNA. Abbreviations used in Table 3: N, normal tissue; T, tumor; Met, metastasis; HMVEC, human vascular endothelial cells; prol, proliferating.

**Table 4**

| **Cell Line** | **32144.1 Mean** | **B-2 Mean** | **Relative Expression** |
|---|---|---|---|
| MCF-7 Breast T | 25.21 | 18.88 | 12.43 |
| ZR75 Breast T | 26.34 | 20.29 | 15.09 |
| T47D Bre a st T | 25.98 | 18.00 | 3.96 |
| M DA 231 Bre a st T | 33.38 | 17.35 | 0.01 |
| M DA 435 Breast T | 30.20 | 15.89 | 0.05 |
| SKBr3 Breast | 28.63 | 18.99 | 1.25 |
| OLD 1 ColonT(stageC) | 24.36 | 19.44 | 33.03 |
| SW620 Colon T (stage C) | 25.12 | 18.26 | 8.58 |
| HC T116 | 25.33 | 18.00 | 6.22 |
| HT29 | 25.09 | 15.88 | 1.68 |
| Colo 205 | 24.23 | 14.83 | 1.48 |
| NC IH125 | 30.26 | 17.38 | 0.13 |
| NC IH322 | 25.66 | 18.36 | 6.37 |
| NC IH460 | 32.71 | 17.33 | 0.02 |
| A549 | 32.39 | 18.62 | 0.07 |
| NHBE | 30.14 | 21.37 | 2.29 |
| SKOV-3 ovary | 28.29 | 17.24 | 0.47 |
| OVCAR-3 ovary | 26.55 | 20.31 | 13.23 |
| 293 baby kidney | 27.04 | 20.28 | 9.23 |
| 293Tbaby kidney | 32.24 | 21.60 | 0.63 |

Table 4 depicts the relative expression of 32144 mRNA in cell lines that have been xenographed into mice and allowed to form tumors. Several of the lines display high levels of 32144 expression when grown under such conditions. Most notable is one of the Stage C colon tumor lines, a couple of the breast tumor lines, one of the ovary carcinoma lines, and a baby kidney fibroblast line. Many of the other cell lines also express 32144 mRNA when xenographed into mice. Abbreviation used in Table 4: T, tumor; HCT116, HT29, and Colo 205, colon carcinoma cell lines; NCIH125, NCIH322, NCIH460, A549, and NHBE, lung carcinoma cell lines.

**Table 5**

| **Tissue Type** | **32144.1 Mean** | **β 2 Mean** | **Relative Expression** |
|---|---|---|---|
| MCF10MS | 31.31 | 19.84 | 0.35 |
| MCF10A | 37.33 | 19.75 | 0.00 |
| MCF10AT.cl1 | 39.96 | 19.48 | 0.00 |
| MCF10AT.cl3 | 38.22 | 18.86 | 0.00 |
| MCF10AT1 | 31.68 | 19.94 | 0.29 |
| MCF10AT3B | 39.88 | 19.47 | 0.00 |
| MCF10CAla.cl1 | 34.26 | 17.09 | 0.01 |
| MCF1 OAT3B Agar | 32.53 | 25.9 | 10.10 |
| MCF10CA1a.cl1 Agar | 33.95 | 24.5 | 1.43 |
| MCF10A.m25 Plastic | 37.11 | 24.54 | 0.00 |
| MCF10CA Agar | 33.07 | 21.5 | 0.33 |
| MCF10CA Plastic | 33.27 | 21.56 | 0.30 |
| MCF3B Agar | 29.58 | 21.84 | 4.68 |
| MCF3B Plastic | 30.32 | 21.58 | 2.35 |
| MCF10A EGF 0 hr | 32.59 | 17.23 | 0.02 |
| MCF10A EGF 0.5 hr | 32.2 | 17.45 | 0.04 |
| MCF10A EGF 1 hr | 32.63 | 17.6 | 0.03 |
| MCF10A EGF 2 hr | 32.83 | 17.63 | 0.03 |
| MCF10A EGF 4 hr | 33.52 | 17.63 | 0.02 |
| MCF10A EGF 8 hr | 33.31 | 17.52 | 0.02 |
| MCF10A IGF1A 0 hr | 31.05 | 21.58 | 1.41 |
| MCF10A IGF1A 0.5 hr | 31.36 | 21.75 | 1.27 |
| MCF10A IGF1A 1 hr | 30.93 | 21.84 | 1.84 |
| MCF10A IGF1A 3 hr | 30.78 | 21.88 | 2.09 |
| MCF10A IGF1A 24 hr | 29.32 | 21.84 | 5.62 |
| MCF10AT3B.cl5 Plastic | 35.42 | 21.82 | 0.00 |
| MCF10AT3B.cl6 Plastic | 35.7 | 21.85 | 0.00 |
| MCF10AT3B.cl3 Plastic | 36.19 | 21.63 | 0.00 |
| MCF10AT3B.cl1 Plastic | 35.02 | 21.72 | 0.00 |
| MCF10AT3B.cl4 Plastic | 35.09 | 21.47 | 0.00 |
| MCFIOAT3B.cl2 Plastic | 36.45 | 21.84 | 0.00 |
| MCF10AT3B.cl5 Agar | 31.91 | 24.06 | 4.33 |
| MCFIOAT3B.c16 Agar | 32.32 | 24.05 | 3.23 |
| MCF-7 | 30.1 | 23.27 | 8.76 |
| ZR--75 | 28.29 | 21.59 | 9.65 |
| T47D | 29.61 | 21.64 | 3.97 |
| MDA-231 | 36.9 | 20.45 | 0.00 |
| MDA-435 | 36.16 | 20.55 | 0.00 |
| SkBr3 | 30.81 | 20.93 | 1.06 |
| Hs578Bst | 36.78 | 19.85 | 0.00 |
| Hs578T | 38.49 | 19.66 | 0.00 |

Table 5 depicts the relative expression of 32144 mRNA in breast carcinoma cell lines grown under various conditions. Growth of the cell lines on agar correlates with an increase in 32144 expression, as shown by the MCF10AT3B, MCF3B, MCF10AT3B clone 5 and MCT10AT3B clone 6 cell lines. MCF10A cells did not display a change in 32144 expression in response to epidermal growth factor (EGF), while they did respond to insulin growth factor lea (IGF-1A) by gradually increasing 32144 mRNA expression over the course of 24 hours.

**Table 6**

| **Spectrum** | **Tissue** | **Expression** |
|---|---|---|
| CHT 800 | Lung - PD-NSC | +/- |
| CHT 813 | Lung - MD-SCC | -/- |
| CHT 726 | Lung - MD-SCC | +/- |
| CHT 331 | Lung - MD-AC | -/- |
| MPI 216 | Lung - Normal | -/- |

| (LUNG: 0/1 normals; 2/4 tumors) | | |
|---|---|---|
| MDA 28 | Ovary - Malignant | -/- |
| MDA 300 | Ovary - MD-AC | +/- |
| MDA 202 | Ovary - Normal | -/- |

| (OVARY: 0/1 normals; 1/2 tumors) | | |
|---|---|---|
| NDR 7 | Breast - IDC | +/- |
| NDR 12 | Breast - IDC | +++/+ |
| NDR 57 | Breast - PD-Ductal AC | +/- |
| CLN 662 | Breast - IDC/IDL | +/- |
| MDA 156 | Breast - DCIS | ++/+ |
| CLN 156 | Breast - PD-IDC | +/+ |
| MDA 91 | Breast - AC | +++/+ |
| PIT 58 | Breast - PD-AC(lung) | -/- |
| CHT 1841 | Breast - Met AC(lymph) | +/- |
| PIT 116 | Breast - Met AC(lung) | +/- |
| MDA 405 | Breast - normal | -/- |
| (BREAST: 0/1 normals; 7/7 tumors; 2/3 metastasis) | | |
| CLN 609 | Colon - Invasive | -/- |
| NDR 99 | Colon - Invasive | +/- |
| NDR 100 | Colon - AC(liver) | +/+ |
| CHT 1 | Colon - Met AC | ++/+ |
| (Colon: 1/2 tumors; 2/2 metastasis) | | |

Expression of 32144 mRNA was analyzed by in-situ hybridization in both normal and tumor tissue samples. Expression of 32144 mRNA was consistently observed in the tumors, suggesting a role for 32144 in tumor development. Furthermore, in colon tumor samples, expression of 32144 mRNA was more prevalent in metastatic tumors, indicating a possible link between 32144 expression and tumor metastasis in some tissues. Abbreviations used in Table 6 include: PD, poorly differentiated; MD, moderately differentiated; NSCC, non-small cell carcinoma; SCC, squamous cell carcinoma; AC, adenocarcinoma; IDC, intvasive ductal carcinoma; ILC, invasive lobular carcinoma; Met, metastasis. Parenthesis indicates the tissue in which the tumor was found, if other than the tissue of origin.

**Table 7**

| **Spectrum** | **Tissue** | **Relative Expression** |
|---|---|---|
| MPM51 | Breast - IIC | 1.29 |
| MPM66 | Breast - IIC | 2.25 |
| MPM67 | Breast - IIC | 1.80 |
| MPM81 | Breast - MetC | 2.50 |
| MPM50 | Breast - MetC | 1.24 |
| MPM68 | Breast - MetC | 10.23 |
| MPM70 | Breast - MetC | 10.22 |
| MPM71 | Breast - MetC | 1.92 |

Expression array-based analysis of human 32144 mRNA expression in invasive indolent breast carcinomas (IIC) and metastatic breast carcinomas (MetC). 2/5 metastatic breast carcinomas displayed an elevated level of 32144 expression, while 0/3 invasive indolent breast carcinomas displayed an elevation in 32144 expression, suggesting a correlation between elevated 32144 expression and tumor metastasis.

### Example 3: Tissue Distribution of 32144 mRNA by Northern Analysis

Northern blot hybridizations with various RNA samples can be performed under standard conditions and washed under stringent conditions, i.e., 0.2xSSC at 65°C. A DNA probe corresponding to all or a portion of the 32144 cDNA (SEQ ID NO:1) can be used. The DNA was radioactively labeled with ³²P-dCTP using the Prime-It Kit (Stratagene, La Jolla, CA) according to the instructions of the supplier. Filters containing mRNA from mouse hematopoietic and endocrine tissues, and cancer cell lines (Clontech, Palo Alto, CA) can be probed in ExpressHyb hybridization solution (Clontech) and washed at high stringency according to manufacturer's recommendations.

### Example 4: Recombinant Expression of 32144 in Bacterial Cells

In this example, 32144 is expressed as a recombinant glutathione-S-transferase (GST) fusion polypeptide in *E. coli* and the fusion polypeptide is isolated and characterized. Specifically, 32144 is fused to GST and this fusion polypeptide is expressed in *E. coli,* e.g., strain PEB199. Expression of the GST-32144 fusion protein in PEB199 is induced with IPTG. The recombinant fusion polypeptide is purified from crude bacterial lysates of the induced PEB 199 strain by affinity chromatography on glutathione beads. Using polyacrylamide gel electrophoretic analysis of the polypeptide purified from the bacterial lysates, the molecular weight of the resultant fusion polypeptide is determined.

### Example 5: Expression of Recombinant 32144 Protein in COS Cells

To express the 32144 gene in COS cells (e.g., COS-7 cells, CV-1 origin SV40 cells; Gluzman (1981) *Celll*23:175-182), the pcDNA/Amp vector by Invitrogen Corporation (San Diego, CA) is used. This vector contains an SV40 origin of replication, an ampicillin resistance gene, an *E*. *coli* replication origin, a CMV promoter followed by a polylinker region, and an SV40 intron and polyadenylation site. A DNA fragment encoding the entire 32144 protein and an HA tag (Wilson et al. (1984) Cell 37:767) or a FLAG tag fused in-frame to its 3' end of the fragment is cloned into the polylinker region of the vector, thereby placing the expression of the recombinant protein under the control of the CMV promoter.

To construct the plasmid, the 32144 DNA sequence is amplified by PCR using two primers. The 5' primer contains the restriction site of interest followed by approximately twenty nucleotides of the 32144 coding sequence starting from the initiation codon; the 3' end sequence contains complementary sequences to the other restriction site of interest, a translation stop codon, the HA tag or FLAG tag and the last 20 nucleotides of the 32144 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 32144_gene is inserted in the correct orientation. The ligation mixture is transformed into E. *coli* cells (strains HB101, DH5α, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the 32144-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. (1989) Molecular Cloning: A Laboratory Manual. 2nd, ed, Cold Spring Harbor Laboratory, Cold Spring Harbor Laboratory Press, Cold Spring Harbor, NY. The expression of the 32144 polypeptide is detected by radiolabelling (³⁵S-methionine or ³⁵S-cysteine available from NEN, Boston, MA, can be used) and immunoprecipitation (Harlow, E. and Lane, D. (1988) Antibodies: A Laboratory Manual, Cold Spring Harbor Laboratory Press, Cold Spring Harbor, NY) using an HA specific monoclonal antibody. Briefly, the cells are labeled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 32144 coding sequence is cloned directly into the polylinker of the pCDNA/Amp vector using the appropriate restriction sites. The resulting plasmid is transfected into COS cells in the manner described above, and the expression of the 32144 polypeptide is detected by radiolabelling and immunoprecipitation using a 32144 specific monoclonal antibody.

## Claims

1. A method for identifying a subject having a lung, colon or ovarian cancer, or at risk of developing a lung, colon or ovarian cancer, the method comprising:
a) contacting a first sample obtained from said subject comprising nucleic acid molecules with a nucleic acid probe that specifically hybridizes to a nucleic acid molecule of SEQ ID NO: 1 or SEQ ID NO:3 as shown in example 1;
b) detecting the presence of a nucleic acid molecule in the first sample that specifically hybridizes to the probe;
c) contacting a second sample from a control subject comprising nucleic acid molecules with a nucleic acid probe which specifically hybridizes to a nucleic acid molecule of SEQ ID NO: 1 or SEQ ID NO:3 as shown in example 1;
d) detecting the presence of a nucleic acid molecule in the second sample that specifically hybridizes to the probe;
e) comparing the level of nucleic acids which hybridise to the probe in the first sample with the level of nucleic acids which hybridize to the probe in the second sample,
wherein an increased level of nucleic acids which hybridise in the first sample relative to the second sample identifies a subject having a lung, colon or ovarian cancer, or at risk of developing a lung, colon or ovarian cancer.

2. The method of claim 1, wherein said hybridization probe is detectably labeled.

3. The method of claim 1, wherein said detecting is by *in situ* hybridization.

4. A method for identifying a subject having a lung, colon or ovarian cancer, or at risk of developing a lung, colon or ovarian cancer, the method comprising:
a) contacting a first sample obtained from said subject comprising nucleic acid molecules with a first and a second amplification primer, each of which specifically hybridizes to a nucleic acid molecule selected from the group consisting of:
i) a nucleic acid molecule consisting of the nucleotide sequence set forth in SEQ ID NO:1 or 3 as shown in example 1; and
ii) a nucleic acid molecule which encodes a polypeptide comprising the amino acid sequence set forth in SEQ ID NO:2 as shown in example 1;
b) incubating the first sample under conditions that allow nucleic acid amplification;
c) contacting a second sample obtained from a control subject comprising nucleic acid molecules with said first and a second amplification primer;
d) incubating the second sample under conditions that allow nucleic acid amplification; and
e) comparing the level of nucleic acid amplification in the first sample relative to the level of nucleic acid amplification in the second sample,
wherein an increased level of nucleic acid amplification in the first sample relative to the second sample identifies a subject having a lung, colon or ovarian cancer, or at risk of developing a lung, colon or ovarian cancer.

5. A method of identifying a subject having a lung, colon or ovarian cancer, or at risk of developing a lung, colon or ovarian cancer comprising:
a) contacting a first sample obtained from said subject comprising polypeptides with an antibody which specifically binds to a polypeptide selected from the group consisting of:
i) a polypeptide comprising the amino acid sequence of SEQ ID NO:2 as shown in example 1; and
ii) a polypeptide which is encoded by a nucleic acid molecule comprising the nucleotide sequence of SEQ ID NO: 1 or 3 as shown in example 1;
b) detecting the presence of a polypeptide in the first sample that binds to the antibody;
c) contacting a second sample from a control subject comprising polypeptides with the antibody;
d) detecting the presence of a polypeptide in the second sample that binds to the antibody; and
e) comparing the level of antibody binding in the first sample relative to the level of antibody binding in the second sample,
wherein an increased level of antibody binding in the first sample relative to the second sample identifies a subject having a lung, colon or ovarian cancer, or at risk of developing a lung, colon or ovarian cancer.

6. The method of claim 5, wherein said antibody is detectably labeled.

7. A method for identifying a compound capable of treating a lung, colon or ovarian cancer, the method comprising:
assaying the ability of the compound to decrease expression of a nucleic acid molecule selected from the group consisting of:
i) a nucleic acid molecule comprising a nucleotide sequence which is at least 95% identical to the nucleotide sequence of SEQ ID NO:1 or 3 as shown in example 1, or a complement thereof;
ii) a nucleic acid molecule which encodes a polypeptide comprising an amino acid sequence which is at least 95% identical to the amino acid sequence of SEQ ID NO:2 as shown in example 1;
iii) a nucleic acid molecule consisting of the nucleotide sequence set forth in SEQ ID NO: 1 or 3 as shown in example 1; and
iv) a nucleic acid molecule which encodes a polypeptide comprising the amino acid sequence set forth in SEQ ID NO:2 as shown in example 1;
thereby identifying a compound capable of treating lung, colon or ovarian cancer.

8. A method for identifying a compound capable of treating a lung, colon or ovarian cancer, the method comprising:
assaying the ability of the compound to decrease the fatty acid amide hydrolase activity of a polypeptide selected from the group consisting of:
i) a polypeptide which is encoded by a nucleic acid molecule comprising a nucleotide sequence which is at least 95% identical to a nucleic acid comprising the nucleotide sequence of SEQ ID NO:1 or 3 as shown in example 1;
ii) a polypeptide comprising an amino acid sequence which is at least 95% identical to the amino acid sequence of SEQ ID NO:2 as shown in example 1;
iii) a polypeptide comprising the amino acid sequence of SEQ ID NO:2 as shown in example 1; and
iv) a polypeptide which is encoded by a nucleic acid molecule comprising the nucleotide sequence of SEQ ID NO:1 or 3 as shown in example 1;
thereby identifying a compound capable of treating lung, colon or ovarian cancer.

9. A method according to claim 8, wherein said assay is a cell based assay.

10. A method according to claim 8, wherein said assay is a cell free assay.

11. A method for evaluating the efficacy of a treatment of a lung, colon or ovarian cancer, in a sample isolated from a subject being treated with a protocol under evaluation, which comprises:
assessing the expression level of a nucleic acid molecule selected from the group consisting of:
a) a nucleic acid molecule comprising a nucleotide sequence which is at least 95% identical to the nucleotide sequence of SEQ ID NO:1 or SEQ ID NO: 3 as shown in example 1;
b) a nucleic acid molecule which encodes a polypeptide comprising the amino acid sequence of SEQ ID NO:2 as shown in example 1; and
c) a nucleic acid molecule comprising the nucleotide sequence of SEQ ID NO:1 or SEQ ID NO:3 as shown in example 1;
wherein a decrease in the expression level of the nucleic acid after the treatment, relative to the level before the treatment, is indicative of the efficacy of the treatment of a lung, colon or ovarian cancer.

12. A method for evaluating the efficacy of a treatment of a lung, colon or ovarian cancer, in a sample isolated from a subject being treated with a protocol under evaluation, which comprises:
assessing the expression level of a polypeptide selected from the group consisting of:
i) a polypeptide which is encoded by a nucleic acid molecule comprising a nucleotide sequence which is at least 95% identical to a nucleic acid comprising the nucleotide sequence of SEQ ID NO: 1 or 3 as shown in example 1;
ii) a polypeptide comprising an amino acid sequence which is at least 95% identical to the amino acid sequence of SEQ ID NO:2 as shown in example 1;
iii) a polypeptide comprising the amino acid sequence of SEQ ID NO:2 as shown in example 1; and
iv) a polypeptide which is encoded by a nucleic acid molecule comprising the nucleotide sequence of SEQ ID NO:1 or 3 as shown in example 1;
wherein a decrease in the expression level of the polypeptide after the treatment, relative to the level before the treatment, is indicative of the efficacy of the treatment of a lung, colon or ovarian cancer.

## Patentansprüche

1. Verfahren zum Identifizieren eines Subjektes mit einem Lungen-, Colon- oder Ovarkrebs, oder bei dem das Risiko besteht, dass er einen Lungen-, Colon- oder Ovarkrebs entwickelt, wobei das Verfahren die folgenden Schritte umfasst:
a) Kontaktieren einer ersten Probe, erhalten von dem Subjekt, umfassend Nukleinsäuremoleküle, mit einer Nukleinsäuresonde, die spezifisch an ein Nukleinsäuremolekül SEQ ID NO: 1 oder SEQ ID NO:3, wie in Beispiel 1 gezeigt, hybridisiert;
b) Detektieren des Vorliegens eines Nukleinsäuremoleküls in der ersten Probe, die spezifisch mit der Sonde hybridisiert;
c) Kontaktieren einer zweiten Probe von einem Kontroll-Subjekt, umfassend Nukleinsäuremoleküle, mit einer Nukleinsäuresonde, die spezifisch an ein Nukleinsäuremolekül SEQ ID NO: 1 oder SEQ ID NO:3, wie sie in Beispiel 1 gezeigt sind, hybridisiert;
d) Detektieren des Vorliegens eines Nukleinsäuremoleküls in der zweiten Probe, die spezifisch mit der Sonde hybridisiert;
e) Vergleichen des Levels der Nukleinsäuren, die mit der Sonde in der ersten Probe hybridisieren, mit dem Level der Nukleinsäuren, die mit der Sonde in der zweiten Probe hybridisieren, wobei ein erhöhtes Level an Nukleinsäuren, die in der ersten Probe relativ zu der zweiten Probe hybridisieren, ein Subjekt mit Lungen-, Colon- oder Ovarkrebs identifiziert, oder ein Subjekt identifiziert, bei dem das Risiko besteht, dass er Lungen-, Colon- oder Ovarkrebs entwickelt.

2. Verfahren von Anspruch 1, wobei die Hybridisierungssonde detektierbar markiert ist.

3. Verfahren von Anspruch 1, wobei das Detektieren mittels *in* s*itu*-Hybridisieren erfolgt.

4. Verfahren zum Identifizieren eines Subjektes mit einem Lungen-, Colon- oder Ovarkrebs, oder bei dem das Risiko besteht, dass er einen Lungen-, Colon- oder Ovarkrebs entwickelt, wobei das Verfahren die folgenden Schritte umfasst:
a) Kontaktieren einer ersten Probe, erhalten von dem Subjekt, umfassend Nukleinsäuremoleküle, mit einem ersten und einen zweiten Amplifikationsprimer, wobei beide jeweils spezifisch an ein Nukleinsäuremolekül ausgewählt aus der Gruppe bestehend aus:
i) ein Nukleinsäuremolekül bestehend aus der Nukleotidsequenz, wie dargestellt in SEQ ID NO: 1 oder 3, wie sie in Beispiel 1 gezeigt sind; und
ii) ein Nukleinsäuremolekül, das für ein Polypeptid kodiert, umfassend die Aminosäuresequenz, wie dargestellt in SEQ ID NO: 2, wie sie in Beispiel 1 gezeigt ist;
b) Inkubieren der ersten Probe unter Bedingungen, die Nukleinsäureamplifikation ermöglichen;
c) Kontaktieren einer zweiten Probe, erhalten von einem Kontroll-Subjekt, umfassend Nukleinsäuremoleküle, mit den erstem und einem zweiten Amplifikationsprimer;
d) Inkubieren der zweiten Probe unter Bedingungen, die Nukleinsäureamplifikation ermöglichen; und
e) Vergleichen des Levels der Nukleinsäureämplifikation in der ersten Probe relativ zu dem Level der Nukleinsäureamplifikation in der zweiten Probe,
wobei ein erhöhtes Level an Nukleinsäureamplifikation in der ersten Probe relativ zu der zweiten Probe ein Subjekt mit einem Lungen-, Colon- oder Ovarkrebs identifiziert, oder ein Subjekt identifiziert, bei dem das Risiko besteht, dass er Lungen-, Colon- oder Ovarkrebs entwickelt.

5. Verfahren zum Identifizieren eines Subjektes mit einem Lungen-, Colon- oder Ovarkrebs, oder bei dem das Risiko besteht, dass er einen Lungen-, Colon- oder Ovarkrebs entwickelt, wobei das Verfahren die folgenden Schritte umfasst:
a) Kontaktieren einer ersten Probe, erhalten von dem Subjekt, umfassend Polypeptide, mit einem Antikörper, der spezifisch an ein Polypeptide bindet ausgewählt aus der Gruppe bestehend aus:
i) ein Polypeptid, umfassend die Aminosäuresequenz SEQ ID NO: 2, wie sie in Beispiel 1 gezeigt ist; und
ii) ein Polypeptid, das durch ein Nukleinsäuremolekül kodiert wird, umfassend die Nukleotidsequenz SEQ ID NO: 1 oder 3, wie sie in Beispiel 1 gezeigt sind;
b) Detektieren des Vorliegens eines Polypeptids in der ersten Probe, das an den Antikörper bindet;
c) Kontaktieren einer zweiten Probe von einem Kontroll-Subjekt, umfassend Polypeptide, mit dem Antikörper;
d) Detektieren des Vorliegens eines Polypeptids in der zweiten Probe, das an den Antikörper bindet;
e) Vergleichen des Levels der Antikörperbindung in der ersten Probe relativ zu dem Level der Antikörperbindung in der zweiten Probe,
wobei ein erhöhtes Level an Antikörperbindung in der ersten Probe relativ zu der zweiten Probe ein Subjekt mit einem Lungen-, Colon- oder Ovarkrebs identifiziert, oder ein Subjekt, bei dem das Risiko besteht, dass er einen Lungen-, Colon- oder Ovarkrebs entwickelt.

6. Verfahren von Anspruch 5, wobei der Antikörper detektierbar markiert ist.

7. Verfahren zum Identifizieren einer Verbindung, die in der Lage ist eine Lungen-, Colon-oder Ovarkrebs zu behandeln, wobei das Verfahren umfasst:
Testen der Fähigkeit der Verbindung die Expression eines Nukleinsäuremoleküls zu vermindern ausgewählt aus der Gruppe bestehend aus:
i) ein Nukleinsäuremolekül, umfassend eine Nukleotidsequenz, die mindestens 95% identisch zu der Nukleotidsequenz von SEQ ID NO: 1 oder 3 ist, wie sie in Beispiel 1 gezeigt sind, oder ein Komplement davon;
ii) ein Nukleinsäuremolekül, das für ein Polypeptid kodiert, umfassend eine Aminosäuresequenz, die mindestens 95% identisch ist zu der Aminosäuresequenz SEQ ID NO: 2, wie sie in Beispiel 1 gezeigt ist;
iii) ein Nukleinsäuremolekül bestehend aus der Nukleotidsequenz, wie dargestellt in SEQ ID NO: 1 oder 3, wie sie in Beispiel 1 gezeigt sind; und
iv) ein Nukleinsäuremolekül, das für ein Polypeptid kodiert, umfassend die Aminosäuresequenz, wie dargestellt in SEQ ID NO: 2, wie sie in Beispiel 1 gezeigt ist;
**dadurch** Identifizieren einer Verbindung, die in der Lage ist Lungen-, Colon-oder Ovarkrebs zu behandeln.

8. Verfahren zum Identifizieren einer Verbindung, die in der Lage ist eine Lungen-, Colon-oder Ovarkrebs zu behandeln, wobei das Verfahren umfasst:
Testen der Fähigkeit der Verbindung die Fettsäureamid-Hydrolyse-Aktivität eines Polypeptids zu vermindern ausgewählt aus der Gruppe bestehend aus:
i) ein Polypeptid, das durch ein Nukleinsäuremolekül kodiert wird, umfassend eine Nukleotidsequenz, die mindestens 95% identisch ist zu einer Nukleinsäure umfassend die Nukleinsäuresequenz SEQ ID NO: 1 oder 3, wie sie in Beispiel 1 gezeigt sind;
ii) ein Polypeptid, umfassend eine Aminosäuresequenz, die die mindestens 95% identisch ist zu der Aminosäuresequenz SEQ ID NO: 2, wie sie in Beispiel 1 gezeigt ist;
iii) ein Polypeptid, umfassend die Aminosäuresequenz SEQ ID NO: 2, wie sie in Beispiel 1 gezeigt ist; und
iv) ein Polypeptid, das durch ein Nukleinsäuremolekül kodiert wird, umfassend die Nukleotidsequenz SEQ ID NO: 1 oder 3, wie sie in Beispiel 1 gezeigt sind;
**dadurch** Identifizieren einer Verbindung, die in der Lage ist Lungen-, Colon-oder Ovarkrebs zu behandeln.

9. Verfahren gemäß Anspruch 8, wobei der Test ein zellbasierter Test ist.

10. Verfahren gemäß Anspruch 8, wobei der Test ein zellfreier Test ist.

11. Verfahren zum Evaluieren der Wirksamkeit einer Behandlung eines Lungen-, Colon-oder Ovarkrebs in einer Probe, isoliert aus einem Subjekt, das mit einem Protokoll unter Evaluation behandelt wird, das umfasst:
Testen des Expressionslevels eines Nukleinsäuremoleküls ausgewählt aus der Gruppe bestehend aus:
a) ein Nukleinsäuremolekül, umfassend eine Nukleotidsequenz, die mindestens 95% identisch zu der Nukleotidsequenz von SEQ ID NO: 1 oder SEQ ID NO:3 ist, wie sie in Beispiel 1 gezeigt sind;
b) ein Nukleinsäuremolekül, das für ein Polypeptid kodiert, umfassend die Aminosäuresequenz SEQ ID NO: 2, wie sie in Beispiel 1 gezeigt ist; und
c) ein Nukleinsäuremolekül umfassend die Nukleotidsequenz SEQ ID NO: 1 oder SEQ ID NO:3, wie sie in Beispiel 1 gezeigt sind; und
wobei ein Abfall des Expressionslevels der Nukleinsäure nach der Behandlung relativ zu dem Level vor der Behandlung die Wirksamkeit der Behandlung eines Lungen-, Colon-oder Ovarkrebs anzeigt.

12. Verfahren zum Evaluieren der Wirksamkeit einer Behandlung eines Lungen-, Colon-oder Ovarkrebs in einer Probe, isoliert aus einem Subjekt, das mit einem Protokoll unter Evaluation behandelt wird, das umfasst:
Testen des Expressionslevels eines Polypeptids ausgewählt aus der Gruppe bestehend aus:
i) ein Polypeptid, das durch ein Nukleinsäuremolekül kodiert wird, umfassend eine Nukleotidsequenz, die mindestens 95% identisch ist zu einer Nukleinsäure umfassend die Nukleinsäuresequenz SEQ ID NO: 1 oder 3, wie sie in Beispiel 1 gezeigt sind;
ii) ein Polypeptid, umfassend eine Aminosäuresequenz, die die mindestens 95% identisch ist zu der Aminosäuresequenz SEQ ID NO: 2, wie sie in Beispiel 1 gezeigt ist;
iii) ein Polypeptid, umfassend die Aminosäuresequenz SEQ ID NO: 2, wie sie in Beispiel 1 gezeigt ist; und
iv) ein Polypeptid, das durch ein Nukleinsäuremolekül kodiert wird, umfassend die Nukleotidsequenz SEQ ID NO: 1 oder 3, wie sie in Beispiel 1 gezeigt sind;
wobei ein Abfall des Expressionslevels der Polypeptids nach der Behandlung relativ zu dem Level vor der Behandlung die Wirksamkeit der Behandlung eines Lungen-, Colon-oder Ovarkrebs anzeigt.

## Revendications

1. Procédé d'identification d'un sujet ayant un cancer des poumons, du côlon ou des ovaires, ou à risque de développer un cancer des poumons, du côlon ou des ovaires, le procédé comprenant :
a) la mise en contact d'un premier échantillon obtenu à partir dudit sujet comprenant des molécules d'acide nucléique avec une sonde d'acide nucléique qui s'hybride spécifiquement à une molécule d'acide nucléique de SEQ ID N° 1 ou SEQ ID N° 3 telle que représentée dans l'exemple 1 ;
b) la détection de la présence d'une molécule d'acide nucléique dans le premier échantillon qui s'hybride spécifiquement à la sonde ;
c) la mise en contact d'un second échantillon provenant d'un sujet témoin comprenant des molécules d'acide nucléique avec une sonde d'acide nucléique qui s'hybride spécifiquement à une molécule d'acide nucléique de SEQ ID N° 1 ou SEQ ID N° 3 telle que représentée dans l'exemple 1 ;
d) la détection de la présence d'une molécule d'acide nucléique dans le second échantillon qui s'hybride spécifiquement à la sonde ;
e) la comparaison du taux d'acides nucléiques qui s'hybrident à la sonde dans le premier échantillon avec le taux d'acides nucléiques qui s'hybrident à la sonde dans le second échantillon,
un taux accru d'acides nucléiques qui s'hybrident dans le premier échantillon par rapport au second échantillon permettant d'identifier un sujet ayant un cancer des poumons, du côlon ou des ovaires, ou à risque de développer un cancer des poumons, du côlon ou des ovaires.

2. Procédé selon la revendication 1, dans lequel ladite sonde d'hybridation est marquée de façon détectable.

3. Procédé selon la revendication 1, dans lequel ladite détection s'effectue par hybridation *in situ.*

4. Procédé d'identification d'un sujet ayant un cancer des poumons, du côlon ou des ovaires, ou à risque de développer un cancer des poumons, du côlon ou des ovaires, le procédé comprenant :
a) la mise en contact d'un premier échantillon obtenu à partir dudit sujet comprenant des molécules d'acide nucléique avec une première et une seconde amorce d'amplification, dont chacune s'hybride spécifiquement à une molécule d'acide nucléique choisie dans le groupe constitué par :
i) une molécule d'acide nucléique constituée de la séquence nucléotidique établie dans SEQ ID N° 1 ou 3 telle que représentée dans l'exemple 1 ; et
ii) une molécule d'acide nucléique qui code pour un polypeptide comprenant la séquence d'acides aminés établie dans SEQ ID N° 2 telle que représentée dans l'exemple 1 ;
b) l'incubation du premier échantillon dans des conditions qui permettent une amplification d'acide nucléique ;
c) la mise en contact d'un second échantillon obtenu à partir d'un sujet témoin comprenant des molécules d'acide nucléique avec lesdites première et seconde amorces d'amplification ;
d) l'incubation du second échantillon dans des conditions qui permettent une amplification d'acide nucléique ; et
e) la comparaison du taux d'amplification d'acide nucléique dans le premier échantillon par rapport au taux d'amplification d'acide nucléique dans le second échantillon,
un taux accru d'amplification d'acide nucléique dans le premier échantillon par rapport au second échantillon permettant d'identifier un sujet ayant un cancer des poumons, du côlon ou des ovaires, ou à risque de développer un cancer des poumons, du côlon ou des ovaires.

5. Procédé d'identification d'un sujet ayant un cancer des poumons, du côlon ou des ovaires, ou à risque de développer un cancer des poumons, du côlon ou des ovaires, comprenant :
a) la mise en contact d'un premier échantillon obtenu à partir dudit sujet comprenant des polypeptides avec un anticorps qui se lie spécifiquement à un polypeptide choisi dans le groupe constitué par :
i) un polypeptide comprenant la séquence d'acides aminés de SEQ ID N° 2 telle que représentée dans l'exemple 1 ; et
ii) un polypeptide qui est codé par une molécule d'acide nucléique comprenant la séquence nucléotidique de SEQ ID N° 1 ou 3 telle que représentée dans l'exemple 1 ;
b) la détection de la présence d'un polypeptide dans le premier échantillon qui se lie à l'anticorps ;
c) la mise en contact d'un second échantillon provenant d'un sujet témoin comprenant des polypeptides avec l'anticorps ;
d) la détection de la présence d'un polypeptide dans le second échantillon qui se lie à l'anticorps ; et
e) la comparaison du taux d'anticorps se liant dans le premier échantillon par rapport au taux d'anticorps se liant dans le second échantillon,
un taux accru d'anticorps se liant dans le premier échantillon par rapport au second échantillon permettant d'identifier un sujet ayant un cancer des poumons, du côlon ou des ovaires, ou à risque de développer un cancer des poumons, du côlon ou des ovaires.

6. Procédé selon la revendication 5, dans lequel ledit anticorps est marqué de façon détectable.

7. Procédé d'identification d'un composé capable de traiter un cancer des poumons, du côlon ou des ovaires, le procédé comprenant :
l'essai de la capacité du composé à diminuer l'expression d'une molécule d'acide nucléique choisie dans le groupe constitué par :
i) une molécule d'acide nucléique comprenant une séquence nucléotidique qui est au moins à 95 % identique à la séquence nucléotidique de SEQ ID N° 1 ou 3 telle que représentée dans l'exemple 1, ou un complément de celle-ci ;
ii) une molécule d'acide nucléique qui code pour un polypeptide comprenant une séquence d'acides aminés qui est au moins à 95 % identique à la séquence d'acides aminés de SEQ ID N° 2 telle que représentée dans l'exemple 1 ;
iii) une molécule d'acide nucléique constituée de la séquence nucléotidique établie dans SEQ ID N° 1 ou 3 telle que représentée dans l'exemple 1 ; et
iv) une molécule d'acide nucléique qui code pour un polypeptide comprenant la séquence d'acides aminés établie dans SEQ ID N° 2 telle que représentée dans l'exemple 1 ;
permettant ainsi d'identifier un composé capable de traiter un cancer des poumons, du côlon ou des ovaires.

8. Procédé d'identification d'un composé capable de traiter un cancer des poumons, du côlon ou des ovaires, le procédé comprenant :
l'essai de la capacité du composé à diminuer l'activité amide d'acide gras hydrolase d'un polypeptide choisi dans le groupe constitué par :
i) un polypeptide qui est codé par une molécule d'acide nucléique comprenant une séquence nucléotidique qui est au moins à 95 % identique à un acide nucléique comprenant la séquence nucléotidique de SEQ ID N° 1 ou 3 telle que représentée dans l'exemple 1 ;
ii) un polypeptide comprenant une séquence d'acides aminés qui est au moins à 95 % identique à la séquence d'acides aminés de SEQ ID N° 2 telle que représentée dans l'exemple 1 ;
iii) un polypeptide comprenant la séquence d'acides aminés de SEQ ID N° 2 telle que représentée dans l'exemple 1 ; et
iv) un polypeptide qui est codé par une molécule d'acide nucléique comprenant la séquence nucléotidique de SEQ ID N° 1 ou 3 telle que représentée dans l'exemple 1 ;
permettant ainsi d'identifier un composé capable de traiter un cancer des poumons, du côlon ou des ovaires.

9. Procédé selon la revendication 8, dans lequel ledit essai est un essai à base de cellules.

10. Procédé selon la revendication 8, dans lequel ledit essai est un essai exempt de cellules.

11. Procédé d'évaluation de l'efficacité d'un traitement d'un cancer des poumons, du côlon ou des ovaires, dans un échantillon isolé à partir d'un sujet qui est traité par un protocole soumis à évaluation, qui comprend :
l'évaluation du taux d'expression d'une molécule d'acide nucléique choisie dans le groupe constitué par :
a) une molécule d'acide nucléique comprenant une séquence nucléotidique qui est au moins à 95 % identique à la séquence nucléotidique de SEQ ID N° 1 ou SEQ- ID N° 3 telle que représentée dans l'exemple 1 ;
b) une molécule d'acide nucléique qui code pour un polypeptide comprenant la séquence d'acides aminés de SEQ ID N° 2 telle que représentée dans l'exemple 1 ; et
c) une molécule d'acide nucléique comprenant la séquence nucléotidique de SEQ ID N° 1 ou SEQ ID N° 3 telle que représentée dans l'exemple 1 ;
une diminution du taux d'expression de l'acide nucléique après le traitement, par rapport au taux avant le traitement, étant indicative de l'efficacité du traitement d'un cancer des poumons, du côlon ou des ovaires.

12. Procédé d'évaluation de l'efficacité d'un traitement d'un cancer des poumons, du côlon ou des ovaires, dans un échantillon isolé à partir d'un sujet qui est traité par un protocole soumis à évaluation, qui comprend :
l'évaluation du taux d'expression d'un polypeptide choisi dans le groupe constitué par :
i) un polypeptide qui est codé par une molécule d'acide nucléique comprenant une séquence nucléotidique qui est au moins à 95 % identique à un acide nucléique comprenant la séquence nucléotidique de SEQ ID N° 1 ou 3 telle que représentée dans l'exemple 1 ;
ii) un polypeptide comprenant une séquence d'acides aminés qui est au moins à 95 % identique à la séquence d'acides aminés de SEQ ID N° 2 telle que représentée dans l'exemple 1 ;
iii) un polypeptide comprenant la séquence d'acides aminés de SEQ ID N° 2 telle que représentée dans l'exemple 1 ; et
iv) un polypeptide qui est codé par une molécule d'acide nucléique comprenant la séquence nucléotidique de SEQ ID N° 1 ou 3 telle que représentée dans l'exemple 1 ;
une diminution du taux d'expression du polypeptide après le traitement, par rapport au taux avant le traitement, étant indicative de l'efficacité du traitement d'un cancer des poumons, du côlon ou des ovaires.
